(19) Europäisches Patentamt
European Patent Office
Office européen des brevets

(11) EP 4 212 535 B1

(12) **EUROPEAN PATENT SPECIFICATION**

(45) Date of publication and mention of the grant of the patent:
**01.10.2025 Bulletin 2025/40**

(21) Application number: **22864835.8**

(22) Date of filing: **23.06.2022**

(51) International Patent Classification (IPC):
***C09K 11/06*** *(2006.01)* ***C07B 59/00*** *(2006.01)*
***C07D 493/04*** *(2006.01)* ***H10K 85/60*** *(2023.01)*
*H10K 50/11 (2023.01)*

(52) Cooperative Patent Classification (CPC):
**C07B 59/002; C07D 493/04; H10K 85/615; H10K 85/631; H10K 85/654; H10K 85/657; H10K 85/6572; H10K 85/6574;** C07B 2200/05; H10K 50/11

(86) International application number:
**PCT/KR2022/008938**

(87) International publication number:
**WO 2023/033326 (09.03.2023 Gazette 2023/10)**

(54) **COMPOUND AND ORGANIC LIGHT-EMITTING ELEMENT COMPRISING SAME**

VERBINDUNG UND ORGANISCHES LICHTEMITTIERENDES ELEMENT DAMIT

COMPOSÉ ET ÉLÉMENT ÉLECTROLUMINESCENT ORGANIQUE LE COMPRENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.09.2021 KR 20210116104**

(43) Date of publication of application:
**19.07.2023 Bulletin 2023/29**

(73) Proprietor: **LG Chem, Ltd.**
**Seoul 07336 (KR)**

(72) Inventors:
- **LEE, Hojung**
  **Daejeon 34122 (KR)**
- **KIM, Seonwoo**
  **Daejeon 34122 (KR)**
- **KIM, Joo Ho**
  **Daejeon 34122 (KR)**
- **KIM, Hoon Jun**
  **Daejeon 34122 (KR)**
- **LEE, Woochul**
  **Daejeon 34122 (KR)**
- **HA, Jae Seung**
  **Daejeon 34122 (KR)**

(74) Representative: **Hoffmann Eitle**
**Patent- und Rechtsanwälte PartmbB**
**Arabellastraße 30**
**81925 München (DE)**

(56) References cited:
**WO-A1-2021/150090**
**KR-A- 20120 029 751**
**KR-A- 20150 128 583**
**KR-A- 20160 141 360**
**KR-A- 20210 093 625**
**KR-A- 20210 093 625**
**KR-A- 20210 093 788**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

[Technical Field]

This application claims priority to and the benefit of Korean Patent Application No. 10-2021-0116104 filed in the Korean Intellectual Property Office on September 1, 2021.

The present specification relates to a compound and an organic light emitting device including the same.

[Background Art]

In general, an organic light emitting phenomenon refers to a phenomenon in which electric energy is converted into light energy by using an organic material. An organic light emitting device using the organic light emitting phenomenon usually has a structure including a positive electrode, a negative electrode, and an organic material layer interposed therebetween. Here, the organic material layer may have a multi-layered structure composed of different materials in many cases in order to improve the efficiency and stability of the organic light emitting device, and may be composed of, for example, a hole injection layer, a hole transport layer, a light emitting layer, an electron transport layer, an electron injection layer, and the like. In such a structure of the organic light emitting device, if a voltage is applied between the two electrodes, holes are injected from the positive electrode into the organic material layer and electrons are injected from the negative electrode into the organic material layer, and when the injected holes and electrons meet each other, an exciton is formed, and light is emitted when the exciton falls down again to a ground state.

KR 10-2021-0093625 relates to an organic electroluminescent element comprising a cathode, an anode and a light emitting layer.

WO 2021/150090 A1 relates to an organic lightemitting element comprising an anode, a cathode, a lightemitting layer and a hole transport region including two or more organic material layers provided between the lightemitting layer and the anode.

There is a continuous need for developing a new material for the aforementioned organic light emitting device.

[Detailed Description of the Invention]

[Technical Problem]

The present specification provides a compound and an organic light emitting device including the same.

[Technical Solution]

An exemplary embodiment of the present specification provides a compound represented by the following Chemical Formula 1.

[Chemical Formula 1]

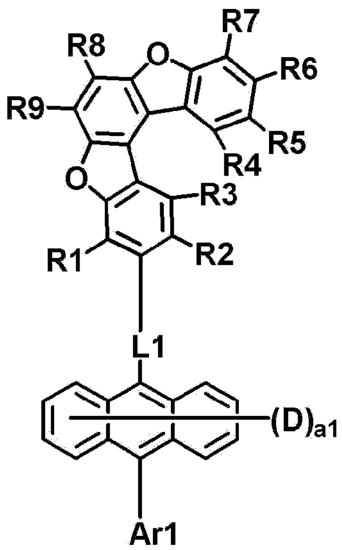

In Chemical Formula 1,

D is deuterium,
L1 is a naphthylene group unsubstituted or substituted with deuterium,
Ar1 is a substituted or unsubstituted aryl group,
R1 to R9 are the same as or different from each other, and are each independently hydrogen; or deuterium, and
a1 is an integer from 1 to 8.

Further, the present specification provides an organic light emitting device including: a first electrode; a second electrode; and an organic material layer having one or more layers provided between the first electrode and the second electrode, in which one or more layers of the organic material layer include the compound.

[Advantageous Effects]

The compound described in the present specification can be used as a material for an organic material layer of an organic light emitting device. The compound according to another exemplary embodiment can improve the efficiency, achieve low driving voltage and/or improve lifetime characteristics in the organic light emitting device.

[Brief Description of Drawings]

FIGS. 1 and 2 illustrate an example of an organic light emitting device according to an exemplary embodiment of the present specification.

[Explanation of Reference Numerals and Symbols]

1: Substrate
2: First electrode
3: Second electrode
4: Light emitting layer
5: First hole injection layer
6: Second hole injection layer
7: Hole transport layer
8: Electron blocking layer
9: First electron transport layer

10: Second electron transport layer
11: Electron injection layer

[Best Mode]

Hereinafter, the present specification will be described in more detail.
An exemplary embodiment of the present specification provides the compound represented by Chemical Formula 1.
Since Chemical formula 1 according to an exemplary embodiment of the present specification includes 1) Ar1 and 2) L1 bound to position 3 of benzobisbenzofuran as substituents at an anthracene core, and has a structure in which at least one hydrogen at a substitutable position of the anthracene of Chemical Formula 1 is substituted with deuterium, there are structural characteristics that the mobility of electrons and holes is improved and the molecular stability is improved. Therefore, an organic light emitting device including the same are excellent in terms of driving voltage, efficiency and service life.
Examples of the substituents in the present specification will be described below, but are not limited thereto.
In the present specification,

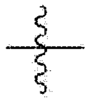

means a moiety to be linked.
Throughout the specification of the present application, the term "combination thereof" included in the Markush type expression means a mixture or combination of one or more selected from the group consisting of constituent elements described in the Markush type expression, and means including one or more selected from the group consisting of the above-described constituent elements.
The term "substitution" means that a hydrogen atom bonded to a carbon atom of a compound is changed into another substituent, and a position to be substituted is not limited as long as the position is a position at which the hydrogen atom is substituted, that is, a position at which the substituent may be substituted, and when two or more are substituted, the two or more substituents may be the same as or different from each other.
In the present invention, the term "substituted or unsubstituted" means being substituted with one or more substituents selected from the group consisting of deuterium; a halogen group; a hydroxyl group; a cyano group; a nitro group; an alkyl group; a cycloalkyl group; an alkoxy group; an alkenyl group; a haloalkyl group; a silyl group; a boron group; an amine group; an aryl group; and a heteroaryl group, being substituted with a substituent to which two or more substituents among the exemplified substituents are linked, or having no substituent.
In the present specification, the fact that two or more substituents are linked indicates that hydrogen of any one substituent is linked to another substituent. For example, when two substituents are linked to each other, a phenyl group and a naphthyl group may be linked to each other to become a substituent of

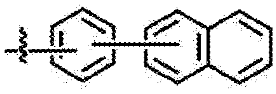

or

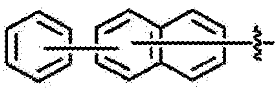

.

Further, the case where three substituents are linked to one another includes not only a case where (Substituent 1)-(Substituent 2)-(Substituent 3) are consecutively linked to one another, but also a case where (Substituent 2) and (Substituent 3) are linked to (Substituent 1). For example, a phenyl group, a naphthyl group, and an isopropyl group may be linked to one another to form a substituent of

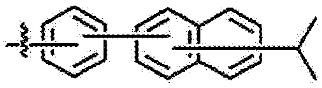,
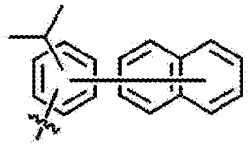,

or

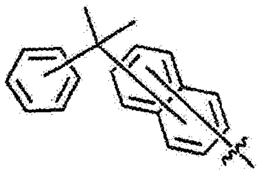.

The above-described definition also applies equally to the case where four or more substituents are linked to one another.

In the present specification, examples of a halogen group include fluorine, chlorine, bromine or iodine.

In the present specification, the alkyl group may be straight-chained or branched, and the number of carbon atoms thereof is not particularly limited, but is preferably 1 to 30. Specific examples thereof include methyl, ethyl, propyl, n-propyl, isopropyl, butyl, n-butyl, isobutyl, tert-butyl, sec-butyl, 1-methyl-butyl, 1-ethylbutyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 2-methylpentyl, 4-methylhexyl, 5-methylhexyl, and the like, but are not limited thereto.

In the present specification, a cycloalkyl group is not particularly limited, but has preferably 3 to 30 carbon atoms, and specific examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, an adamantyl group, and the like, but are not limited thereto.

In the present specification, the alkoxy group may be straight-chained, branched, or cyclic. The number of carbon atoms of the alkoxy group is not particularly limited, but is preferably 1 to 30. Specific examples thereof include methoxy, ethoxy, n-propoxy, isopropoxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy, and the like, but are not limited thereto.

In the present specification, the alkenyl group may be straight-chained or branched, and the number of carbon atoms thereof is not particularly limited, but is preferably 2 to 30. Specific examples thereof include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl) vinyl-1-yl, a stilbenyl group, a styrenyl group, and the like, but are not limited thereto.

In the present specification, the haloalkyl group means that at least one halogen group is substituted instead of hydrogen in an alkyl group in the definition of the alkyl group.

In the present specification, an aryl group is not particularly limited, but has preferably 6 to 30 carbon atoms, and the aryl group may be monocyclic or polycyclic.

When the aryl group is a monocyclic aryl group, the number of carbon atoms thereof is not particularly limited, but is preferably 6 to 30. Specific examples of the monocyclic aryl group include a phenyl group, a biphenyl group, a terphenyl group, and the like, but are not limited thereto.

When the aryl group is a polycyclic aryl group, the number of carbon atoms thereof is not particularly limited, but is preferably 10 to 30. Specific examples of the polycyclic aryl group include a naphthyl group, an anthracene group, a phenanthrene group, a triphenylene group, a pyrene group, a phenalene group, a perylene group, a chrysene group, a fluorene group, and the like, but are not limited thereto.

In the present specification, the fluorene group may be substituted, and adjacent groups may be bonded to each other to form a ring.

Examples of the case where the fluorene group is substituted include

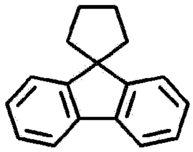 , 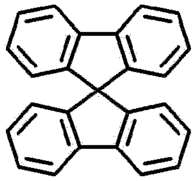 , 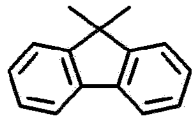 ,

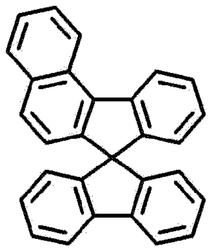 , 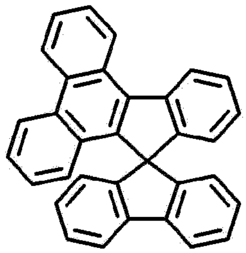 , 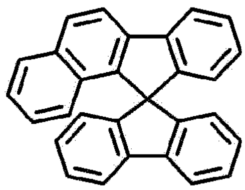 ,

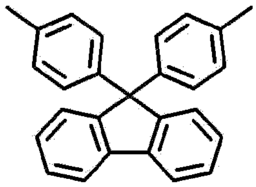 , 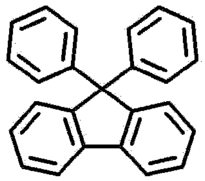 ,

and the like, but are not limited thereto.

In the present specification, the "adjacent" group may mean a substituent substituted with an atom directly linked to an atom in which the corresponding substituent is substituted, a substituent disposed to be sterically closest to the corresponding substituent, or another substituent substituted with an atom in which the corresponding substituent is substituted. For example, two substituents substituted at the ortho position in a benzene ring and two substituents substituted with the same carbon in an aliphatic ring may be interpreted as groups which are "adjacent" to each other.

In the present specification, a heteroaryl group includes one or more atoms other than carbon, that is, one or more heteroatoms, and specifically, the heteroatom may include one or more atoms selected from the group consisting of O, N, Se, S, and the like. The number of carbon atoms thereof is not particularly limited, but is preferably 2 to 30, and the heteroaryl group may be monocyclic or polycyclic. Examples of the heteroaryl group include a thiophene group, a furan group, a pyrrole group, an imidazole group, a thiazole group, an oxazole group, an oxadiazole group, a pyridine group, a bipyridine group, a pyrimidine group, a triazine group, a triazole group, an acridine group, a pyridazine group, a pyrazine group, a quinoline group, a quinazoline group, a quinoxaline group, a phthalazine group, a pyridopyrimidine group, a pyridopyrazine group, a pyrazinopyrazine group, an isoquinoline group, an indole group, a carbazole group, a benzoxazole group, a benzimidazole group, a benzothiazole group, a benzocarbazole group, a benzothiophene group, a dibenzothiophene group, a benzofuran group, a phenanthridine group, a phenanthroline group, an isoxazole group, a thiadiazole group, a dibenzofuran group, a dibenzosilole group, a phenoxathiine group, a phenoxazine group, a phenothiazine group, a dihydroindenocarbazole group, a spirofluorenexanthene group, a spirofluorenethioxanthene group, and the like, but are not limited thereto.

In the present specification, the silyl group may be an alkylsilyl group, an arylsilyl group, a heteroarylsilyl group, and the like. The above-described examples of the alkyl group may be applied to the alkyl group in the alkylsilyl group, the above-described examples of the aryl group may be applied to the aryl group in the arylsilyl group, and the examples of the heteroaryl group may be applied to the heteroaryl group in the heteroarylsilyl group.

In the present specification, a boron group may be $-BR_{100}R_{101}$, and $R_{100}$ and $R_{101}$ are the same as or different from each other, and may be each independently selected from the group consisting of hydrogen; deuterium; halogen; a nitrile group; a substituted or unsubstituted monocyclic or polycyclic cycloalkyl group having 3 to 30 carbon atoms; a substituted or unsubstituted straight-chained or branched alkyl group having 1 to 30 carbon atoms; a substituted or unsubstituted monocyclic or polycyclic aryl group having 6 to 30 carbon atoms; and a substituted or unsubstituted monocyclic or polycyclic heteroaryl group having 2 to 30 carbon atoms. Specific examples of the boron group include a dimethylboron group, a diethylboron group, a t-butylmethylboron group, a diphenylboron group, and the like, but are not limited thereto.

In the present specification, an amine group may be selected from the group consisting of $-NH_2$, an alkylamine

group, an N-alkylarylamine group, an arylamine group, an N-arylheteroarylamine group, an N-alkylheteroarylamine group, and a heteroarylamine group, and the number of carbon atoms thereof is not particularly limited, but is preferably 1 to 30. Specific examples of the amine group include a methylamine group, a dimethylamine group, an ethylamine group, a diethylamine group, a phenylamine group, a naphthylamine group, a biphenylamine group, an anthracenylamine group, a 9-methyl-anthracenylamine group, a diphenylamine group, a ditolylamine group, an N-phenyltolylamine group, an N-phenylbiphenylamine group, an N-phenylnaphthylamine group, an N-biphenylnaphthylamine group, an N-naphthylfluorenylamine group, an N-phenylphenanthrenylamine group, an N-biphenylphenanthrenylamine group, an N-phenylfluorenylamine group, an N-phenyl terphenylamine group, an N-phenanthrenylfluorenylamine group, an N-biphenylfluorenylamine group, and the like, but are not limited thereto.

In the present specification, an N-alkylarylamine group means an amine group in which an alkyl group and an aryl group are substituted with N of the amine group. The alkyl group and the aryl group in the N-alkylarylamine group are the same as the above-described examples of the alkyl group and the aryl group.

In the present specification, an N-arylheteroarylamine group means an amine group in which an aryl group and a heteroaryl group are substituted with N of the amine group. The aryl group and heteroaryl group in the N-arylheteroarylamine group are the same as the above-described examples of the aryl group and the heteroaryl group.

In the present specification, an N-alkylheteroarylamine group means an amine group in which an alkyl group and a heteroaryl group are substituted with N of the amine group. The alkyl group and the heteroaryl group in the N-alkylheteroarylamine group are the same as the above-described examples of the alkyl group and the heteroaryl group.

In the present specification, examples of an alkylamine group include a substituted or unsubstituted monoalkylamine group or a substituted or unsubstituted dialkylamine group. The alkyl group in the alkylamine group may be a straight-chained or branched alkyl group. The alkylamine group including two or more alkyl groups may include a straight-chained alkyl group, a branched alkyl group, or both a straight-chained alkyl group and a branched alkyl group. For example, the alkyl group in the alkylamine group may be selected from the above-described examples of the alkyl group.

In the present specification, examples of an arylamine group include a substituted or unsubstituted monoarylamine group or a substituted or unsubstituted diarylamine group. The aryl group in the arylamine group may be a monocyclic aryl group or a polycyclic aryl group. The arylamine group including two or more aryl groups may include a monocyclic aryl group, a polycyclic aryl group, or both a monocyclic aryl group and a polycyclic aryl group. For example, the aryl group in the arylamine group may be selected from the above-described examples of the aryl group.

In the present specification, examples of a heteroarylamine group include a substituted or unsubstituted monoheteroarylamine group or a substituted or unsubstituted diheteroarylamine group. The heteroarylamine group including two or more heteroaryl groups may include a monocyclic heteroaryl group, a polycyclic heteroaryl group, or both a monocyclic heteroaryl group and a polycyclic heteroaryl group. For example, the heteroaryl group in the heteroarylamine group may be selected from the above-described examples of the heteroaryl group.

In the present specification, an arylene group means a group having two bonding positions in an aryl group, that is, a divalent group. The above-described description on the aryl group may be applied to the arylene group, except that the arylene groups are each a divalent group.

In the present specification, "deuteration" or "deuterated" means that hydrogen at a substitutable position of a compound is substituted with deuterium.

In the present specification, "overdeuterated" means a compound or group in which all hydrogens in the molecule are substituted with deuterium, and has the same meaning as "100% deuterated".

In the present specification, "X% deuterated", "X% degree of deuteration", or "X% deuterium substitution rate" means that X% of the hydrogens at substitutable positions in the corresponding structure are substituted with deuterium. For example, when the corresponding structure is dibenzofuran, the dibenzofuran being "25% deuterated", "25% degree of deuteration" of the dibenzofuran, or "25% deuterium substitution rate" of the dibenzofuran means that 2 of the 8 hydrogens at the substitutable positions of the dibenzofuran are substituted with deuterium.

In the present specification, the degree of deuteration may be confirmed by a known method such as nuclear magnetic resonance spectroscopy ($^1$H NMR), thinlayer chromatography/mass spectrometry (TLC/MS), or matrix assisted laser desorption/ionization time-of-flight mass spectrometry (MALDI-TOF MS).

Unless otherwise defined in the present specification, all technical and scientific terms used in the present specification have the same meaning as commonly understood by one with ordinary skill in the art to which the present invention pertains. Although methods and materials similar to or equivalent to those described in the present specification may be used in the practice or in the test of exemplary embodiments of the present invention, suitable methods and materials will be described below. However, the materials, methods, and examples are illustrative only and are not intended to be limiting to the invention set out in the appended set of claims.

According to an exemplary embodiment of the present specification, Chemical Formula 1 is represented by the following Chemical Formula 1-2.

[Chemical Formula 1-2]

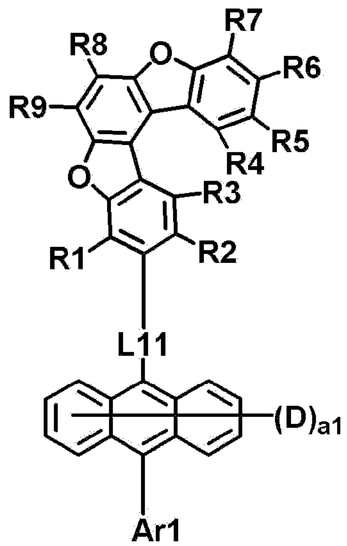

In Chemical Formula 1-2,
the definitions of D, Ar1, a1 and R1 to R9 are the same as those defined in Chemical Formula 1, and L11 is a naphthylene group unsubstituted or substituted with deuterium.

According to an exemplary embodiment of the present specification, Chemical Formula 1 is represented by the following Chemical Formula 1-4.

[Chemical Formula 1-4]

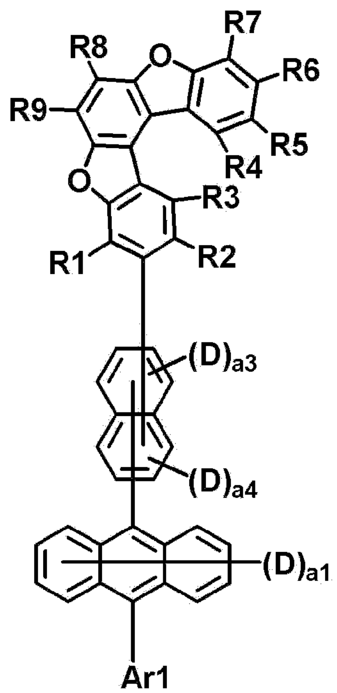

In Chemical Formula 1-4,

the definitions of D, Ar1, a1 and R1 to R9 are the same as those defined in Chemical Formula 1,
a3 is an integer from 0 to 4,

a4 is an integer from 0 to 4, and

$$0 \leq a3 + a4 \leq 6.$$

According to one embodiment of the specification, 0 in the definitions of a3 to a4 means that hydrogen is bound instead of deuterium.

According to an exemplary embodiment of the present specification, a3 is an integer from 1 to 4.

According to an exemplary embodiment of the present specification, a3 is 0.

According to an exemplary embodiment of the present specification, a3 is 1.

According to an exemplary embodiment of the present specification, a3 is 2.

According to an exemplary embodiment of the present specification, a3 is 3.

According to an exemplary embodiment of the present specification, a3 is 4.

According to an exemplary embodiment of the present specification, a4 is an integer from 1 to 4.

According to an exemplary embodiment of the present specification, a4 is 0.

According to an exemplary embodiment of the present specification, a4 is 1.

According to an exemplary embodiment of the present specification, a4 is 2.

According to an exemplary embodiment of the present specification, a4 is 3.

According to an exemplary embodiment of the present specification, a4 is 4.

According to an exemplary embodiment of the present specification, a1 is 1.

According to an exemplary embodiment of the present specification, a1 is 2.

According to an exemplary embodiment of the present specification, a1 is 3.

According to an exemplary embodiment of the present specification, a1 is 4.

According to an exemplary embodiment of the present specification, a1 is 5.

According to an exemplary embodiment of the present specification, a1 is 6.

According to an exemplary embodiment of the present specification, a1 is 7.

According to an exemplary embodiment of the present specification, a1 is 8.

According to an exemplary embodiment of the present specification, at least one of R1 to R9 is deuterium, and the others are hydrogen.

According to an exemplary embodiment of the present specification, one of R1 to R9 is deuterium, and the others are hydrogen.

According to an exemplary embodiment of the present specification, two of R1 to R9 are deuterium, and the others are hydrogen.

According to an exemplary embodiment of the present specification, three of R1 to R9 are deuterium, and the others are hydrogen.

According to an exemplary embodiment of the present specification, four of R1 to R9 are deuterium, and the others are hydrogen.

According to an exemplary embodiment of the present specification, five of R1 to R9 are deuterium, and the others are hydrogen.

According to an exemplary embodiment of the present specification, six of R1 to R9 are deuterium, and the others are hydrogen.

According to an exemplary embodiment of the present specification, seven of R1 to R9 are deuterium, and the others are hydrogen.

According to an exemplary embodiment of the present specification, eight of R1 to R9 are deuterium, and the other is hydrogen.

According to an exemplary embodiment of the present specification, R1 to R9 are deuterium.

According to an exemplary embodiment of the present specification, R1 to R9 are hydrogen.

According to an exemplary embodiment of the present specification, Ar1 is a monocyclic or polycyclic aryl group having 6 to 30 carbon atoms, which is unsubstituted or substituted with one or more substituents of deuterium, a monocyclic or polycyclic aryl group having 6 to 30 carbon atoms, and a combination thereof.

According to an exemplary embodiment of the present specification, Ar1 is a substituted or unsubstituted monocyclic or polycyclic aryl group having 6 to 30 carbon atoms.

According to an exemplary embodiment of the present specification, Ar1 is a substituted or unsubstituted monocyclic or polycyclic aryl group having 6 to 20 carbon atoms.

According to an exemplary embodiment of the present specification, Ar1 is a monocyclic or polycyclic aryl group having 6 to 30 carbon atoms, which is unsubstituted or substituted with one or more substituents of deuterium, a monocyclic or polycyclic aryl group having 6 to 30 carbon atoms, and a combination thereof.

According to an exemplary embodiment of the present specification, Ar1 is a monocyclic or polycyclic aryl group

having 6 to 20 carbon atoms, which is unsubstituted or substituted with one or more substituents of deuterium, a monocyclic or polycyclic aryl group having 6 to 20 carbon atoms, and a combination thereof.

According to an exemplary embodiment of the present specification, Ar1 is a phenyl group unsubstituted or substituted with one or more substituents of deuterium, a naphthyl group, and a combination; a biphenyl group unsubstituted or substituted with deuterium; or a naphthyl group unsubstituted or substituted with deuterium.

According to an exemplary embodiment of the present specification, Chemical Formula 1 is any one selected from the following compounds.

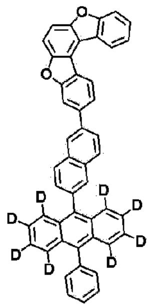
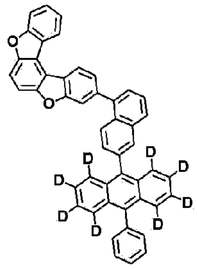
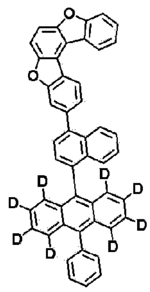

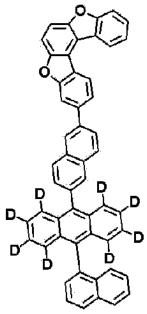
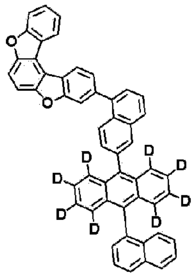
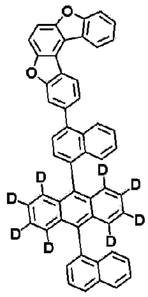

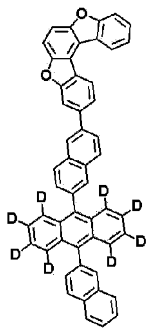
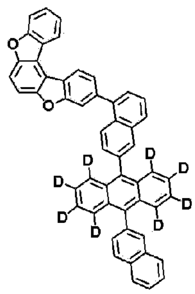
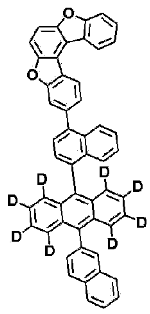

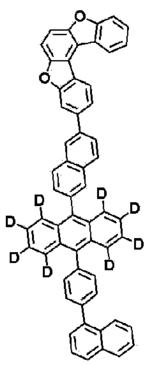
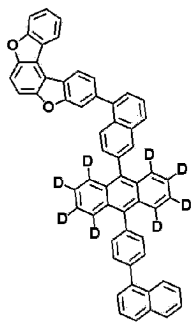
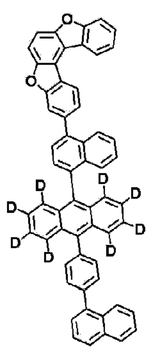

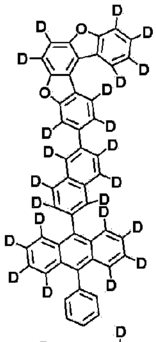
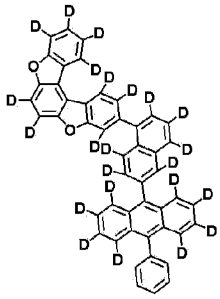
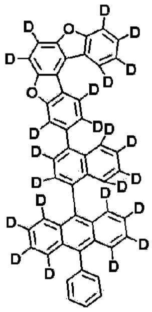

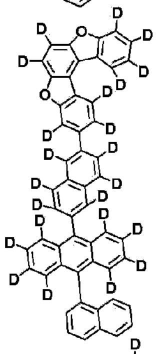
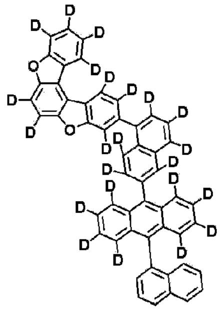
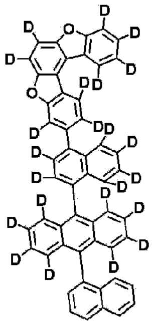

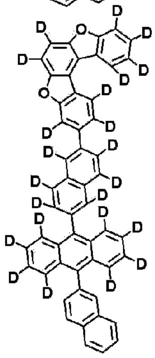
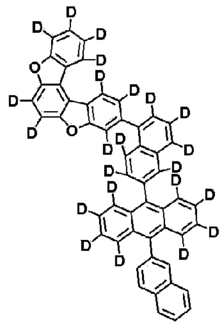
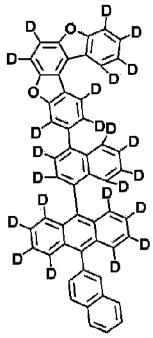

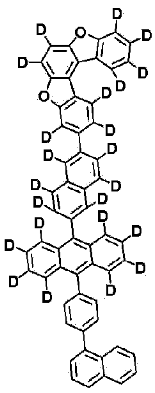
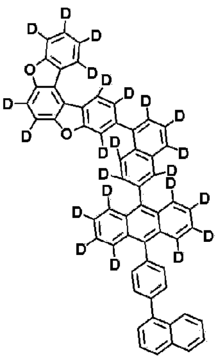
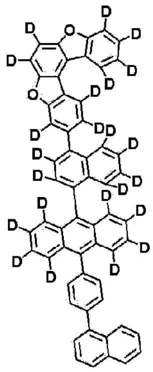

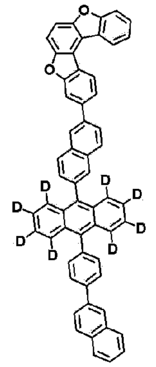
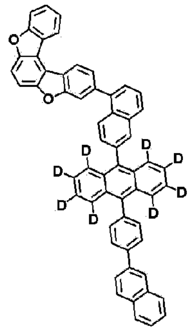
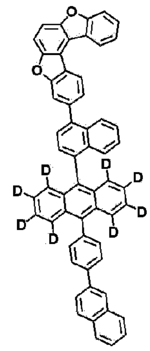

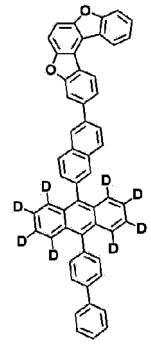
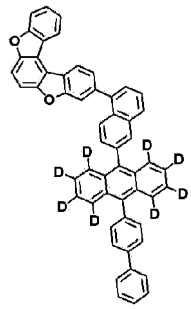
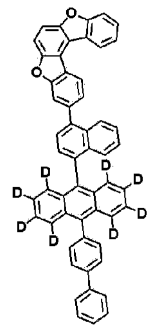

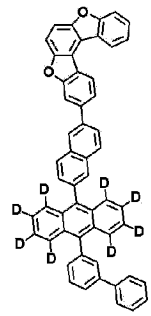
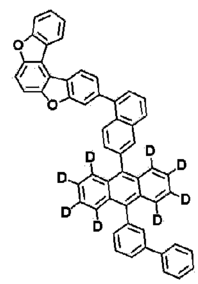
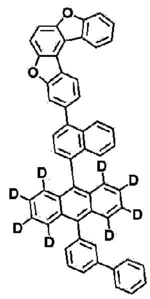

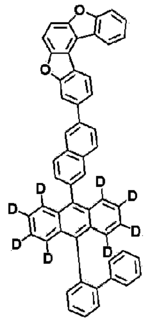
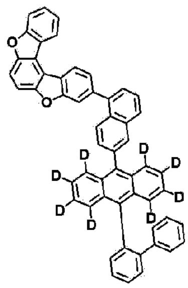
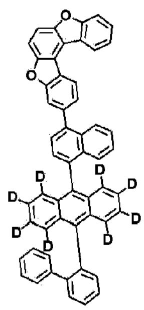

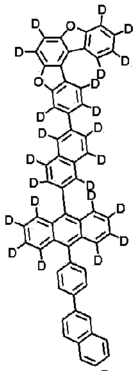
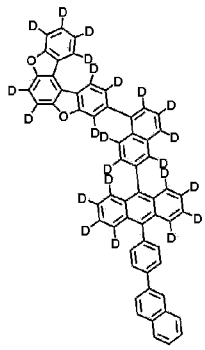
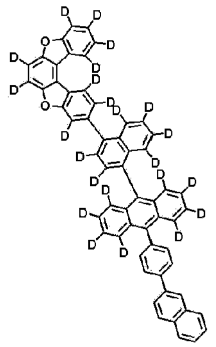

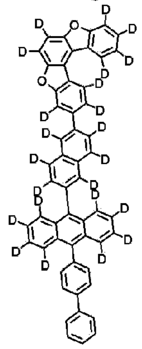
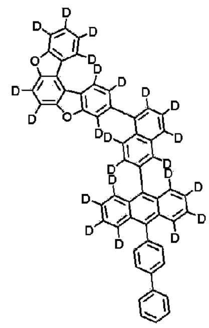
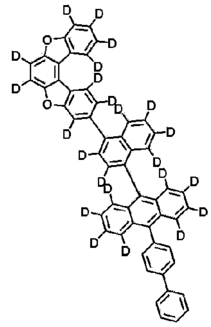

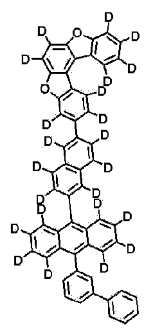
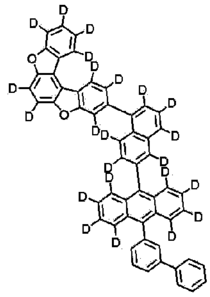
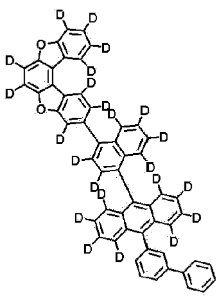

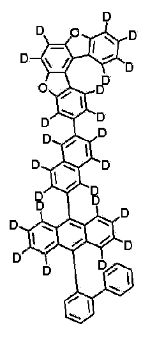
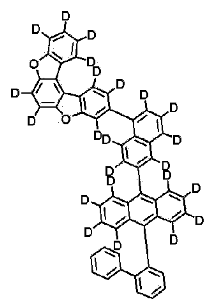
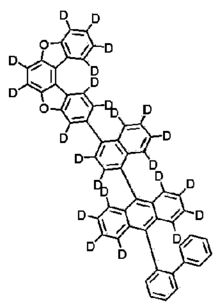

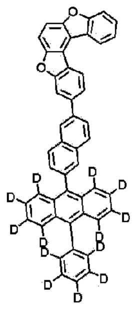
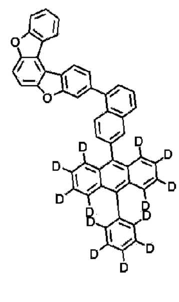
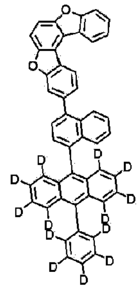

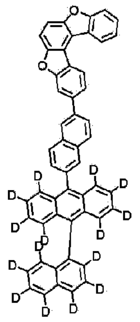

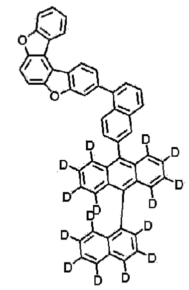

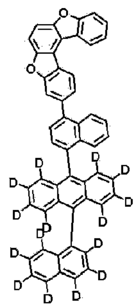

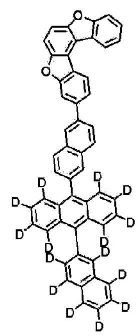

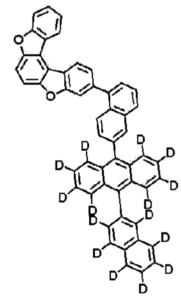

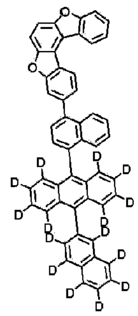

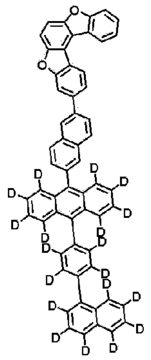

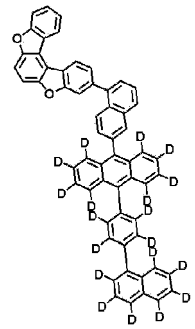

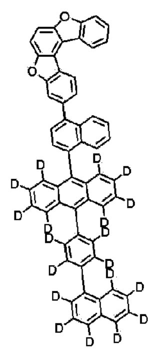

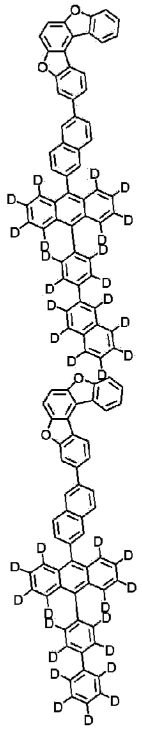
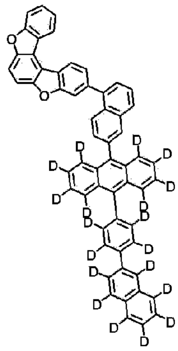
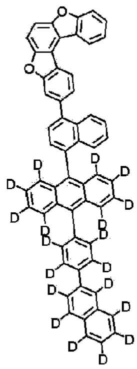
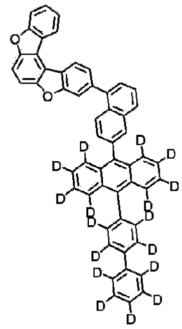
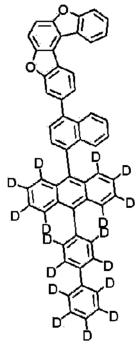

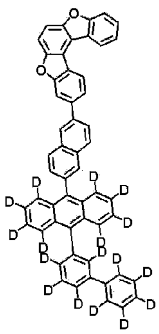
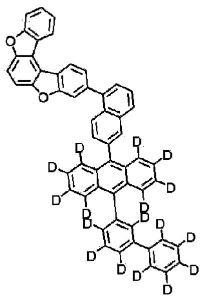
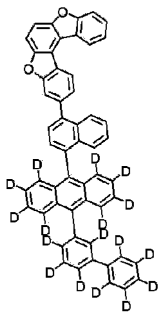

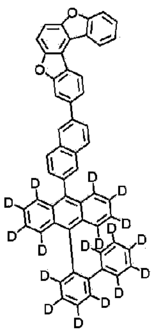
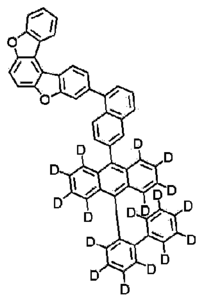
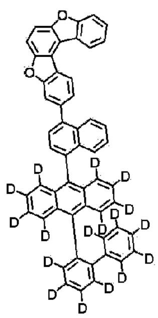

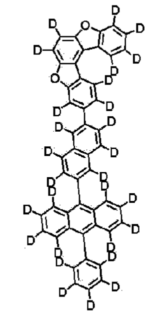

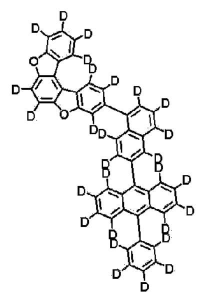

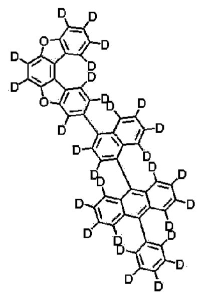

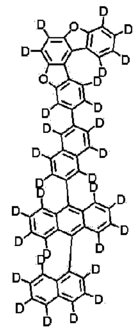

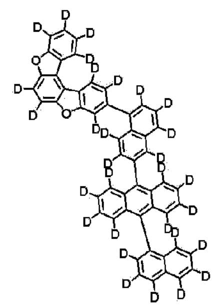

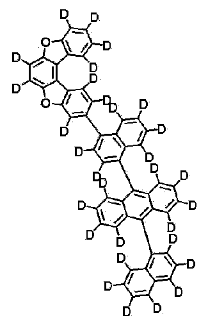

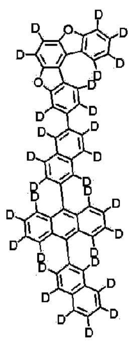

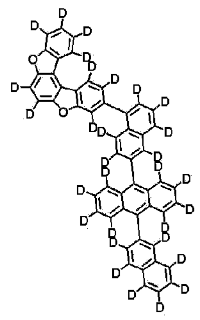

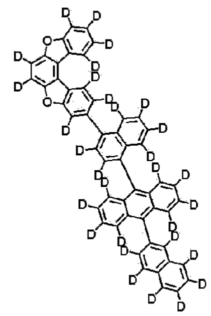

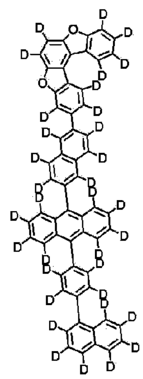

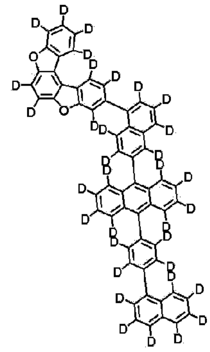

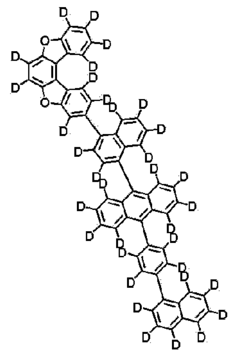

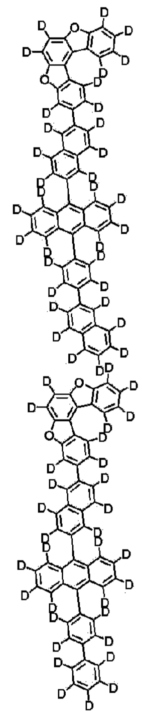
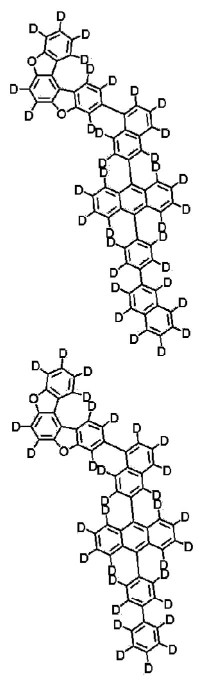
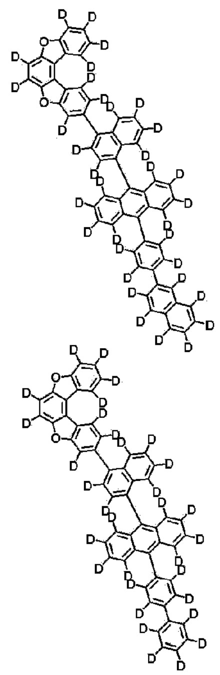

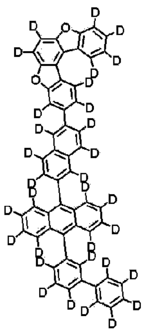
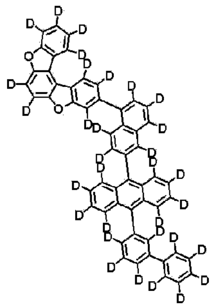
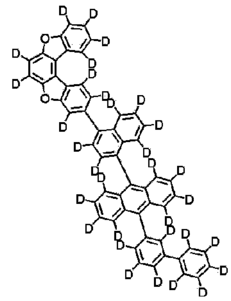

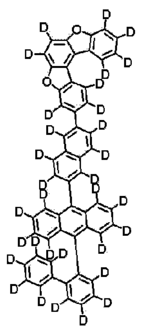
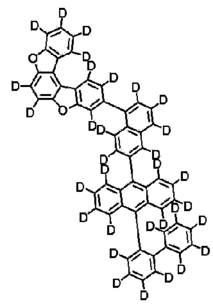
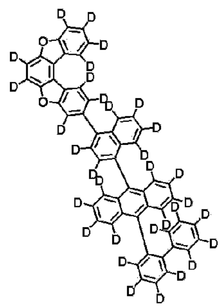

The present specification provides an organic light emitting device including the above-described compound.

When one member is disposed "on" another member in the present specification, this includes not only a case where the one member is brought into contact with another member, but also a case where still another member is present between the two members.

When one part "includes" one constituent element in the present specification, unless otherwise specifically described, this does not mean that another constituent element is excluded, but means that another constituent element may be further included.

In the present specification, the 'layer' has a meaning compatible with a 'film' usually used in the art, and means a coating covering a target region. The size of the 'layer' is not limited, and the sizes of the respective 'layers' may be the same as or different from one another. According to an exemplary embodiment, the size of the 'layer' may be the same as that of the entire device, may correspond to the size of a specific functional region, and may also be as small as a single sub-pixel.

In the present specification, when a specific A material is included in a B layer, this means both i) the fact that one or more A materials are included in one B layer and ii) the fact that the B layer is composed of one or more layers, and the A material is included in one or more layers of the multi-layered B layer.

In the present specification, when a specific A material is included in a C layer or a D layer, this means all of i) the fact that the A material is included in one or more layers of the C layer having one or more layers, ii) the fact that the A material is included in one or more layers of the D layer having one or more layers, and iii) the fact that the A material is included in each of the C layer having one or more layers and the D layer having one or more layers.

The present specification provides an organic light emitting device including: a first electrode; a second electrode; and an organic material layer having one or more layers provided between the first electrode and the second electrode, in which one or more layers of the organic material layer include the compound represented by Chemical Formula **1.**

The organic material layer of the organic light emitting device of the present specification may also be composed of a single-layered structure, but may be composed of a multi-layered structure in which two or more organic material layers are stacked. For example, the organic material layer may have a structure including a hole injection layer, a hole transport layer, a light emitting layer, an electron transport layer, an electron injection layer, an electron blocking layer, a hole blocking layer, and the like. However, the structure of the organic light emitting device is not limited thereto, and may include a fewer number of organic layers.

According to an exemplary embodiment of the present specification, the organic material layer includes a hole injection layer, a hole transport layer, or an electron blocking layer, and the hole injection layer, the hole transport layer, or the electron blocking layer includes the compound.

According to an exemplary embodiment of the present specification, the organic material layer includes a light emitting layer, and the light emitting layer includes the compound.

According to an exemplary embodiment of the present specification, the organic material layer includes a light emitting layer, and the light emitting layer includes the compound as a host of the light emitting layer.

According to an exemplary embodiment of the present specification, the light emitting layer includes a dopant, and the dopant includes one or more selected from the group consisting of a fluorescent dopant, a phosphorescent dopant, and a thermally delayed fluorescent dopant.

According to an exemplary embodiment of the present specification, the fluorescent dopant includes one or more selected from the group consisting of an arylamine-based compound and a boron-based compound.

According to an exemplary embodiment of the present specification, the fluorescent dopant is an arylamine-based compound.

According to an exemplary embodiment of the present specification, the fluorescent dopant is a boron-based compound.

According to an exemplary embodiment of the present specification, the organic material layer includes a light emitting layer, the light emitting layer includes a host and a dopant, the host includes the compound, and the dopant includes one or more selected from the group consisting of the fluorescent dopant, the phosphorescent dopant, and the thermally delayed fluorescent dopant.

According to an exemplary embodiment of the present specification, the arylamine-based compound is represented by the following Chemical Formula D-1.

[Chemical Formula D-1]

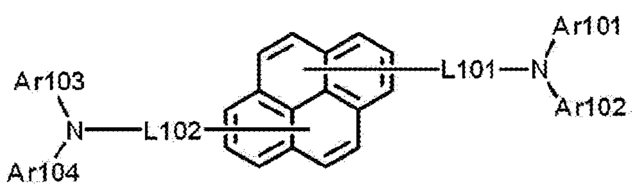

In Chemical Formula D-1,

L101 and L102 are the same as or different from each other, and are each independently a direct bond; or a substituted or unsubstituted arylene group, and

Ar101 to Ar104 are the same as or different from each other, and are each independently a substituted or unsubstituted aryl group; or a substituted or unsubstituted heteroaryl group.

According to an exemplary embodiment of the present specification, L101 and L102 are a direct bond.

According to an exemplary embodiment of the present specification, Ar101 to Ar104 are the same as or different from each other, and are each independently a substituted or unsubstituted monocyclic or polycyclic aryl group having 6 to 30 carbon atoms; or a substituted or unsubstituted monocyclic or polycyclic heteroaryl group having 2 to 30 carbon atoms.

According to an exemplary embodiment of the present specification, Ar101 to Ar104 are the same as or different from each other, and are each independently a monocyclic or polycyclic aryl group having 6 to 30 carbon atoms, which is unsubstituted or substituted with a straight-chained or branched alkyl group having 1 to 30 carbon atoms; or a monocyclic or polycyclic heteroaryl group having 2 to 30 carbon atoms.

According to an exemplary embodiment of the present specification, Ar101 to Ar104 are the same as or different from each other, and are each independently a phenyl group substituted with a methyl group; or a dibenzofuran group.

According to an exemplary embodiment of the present specification, Chemical Formula D-1 is represented by the following compound.

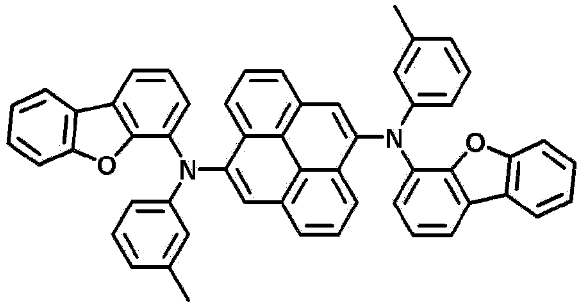

According to an exemplary embodiment of the present specification, the boron-based compound is represented by the following Chemical Formula D-2.

[Chemical Formula D-2]

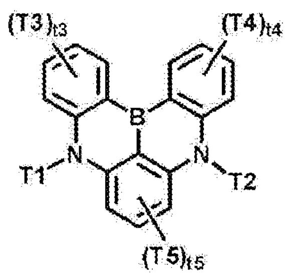

In Chemical Formula D-2,

T1 to T5 are the same as or different from each other, and are each independently hydrogen; a substituted or unsubstituted alkyl group; a substituted or unsubstituted amine group; or a substituted or unsubstituted aryl group,
t3 and t4 are each an integer from 1 to 4,
t5 is an integer from 1 to 3,
when t3 is 2 or higher, two or more T3's are the same as or different from each other,
when t4 is 2 or higher, two or more T4's are the same as or different from each other, and
when t5 is 2 or higher, two or more T5's are the same as or different from each other.

According to an exemplary embodiment of the present specification, T1 to T5 are the same as or different from each other, and are each independently hydrogen; a substituted or unsubstituted straight-chained or branched alkyl group having 1 to 30 carbon atoms; a substituted or unsubstituted monocyclic or polycyclic arylamine group having 6 to 30 carbon atoms; or a substituted or unsubstituted monocyclic or polycyclic aryl group having 6 to 30 carbon atoms.

According to an exemplary embodiment of the present specification, T1 to T5 are the same as or different from each other, and are each independently hydrogen; a straight-chained or branched alkyl group having 1 to 30 carbon atoms; a monocyclic or polycyclic arylamine group having 6 to 30 carbon atoms; or a monocyclic or polycyclic aryl group having 6 to 30 carbon atoms, which is unsubstituted or substituted with a straight-chained or branched alkyl group having 1 to 30 carbon atoms.

According to an exemplary embodiment of the present specification, T1 to T5 are the same as or different from each other, and are each independently hydrogen; a tert-butyl group; a diphenylamine group; or a phenyl group unsubstituted or substituted with a tert-butyl group.

According to an exemplary embodiment of the present specification, Chemical Formula D-2 is represented by the following compound.

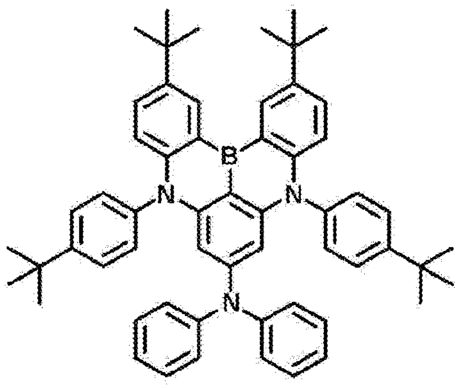

According to an exemplary embodiment of the present specification, as the phosphorescent dopant and the thermally delayed fluorescent dopant, those known in the art are used, but the phosphorescent dopant and the thermally delayed fluorescent dopant are not limited thereto.

According to an exemplary embodiment of the present specification, the light emitting layer includes the host and the dopant at a weight ratio of 99:1 to 1:99. The light emitting layer includes the host and the dopant specifically, at a weight ratio of 99:1 to 50:50, and more specifically, at a weight ratio of 99:1 to 95:5.

According to an exemplary embodiment of the present specification, the organic light emitting device further includes one or two or more layers selected from the group consisting of a hole injection layer, a hole transport layer, a light emitting layer, an electron transport layer, an electron injection layer, a hole blocking layer, and an electron blocking layer.

According to an exemplary embodiment of the present specification, the organic light emitting device includes: a first electrode; a second electrode; a light emitting layer provided between the first electrode and the second electrode; and an organic material layer having two or more layers provided between the light emitting layer and the first electrode, or between the light emitting layer and the second electrode.

According to an exemplary embodiment of the present specification, as the organic material layer having two or more layers between the light emitting layer and the first electrode, or between the light emitting layer and the second electrode, two or more may be selected from the group consisting of a light emitting layer, a hole transport layer, a hole injection layer, a hole injection and transport layer, an electron blocking layer, a hole blocking layer, an electron injection layer, an electron transport layer, and an electron injection and transport layer.

According to an exemplary embodiment of the present specification, a hole injection layer having two or more layers is included between the light emitting layer and the first electrode. The hole injection layer having two or more layers may include materials which are the same as or different from each other.

According to an exemplary embodiment of the present specification, a hole transport layer having two or more layers is included between the light emitting layer and the first electrode. The hole transport layer having two or more layers may include materials which are the same as or different from each other.

According to an exemplary embodiment of the present specification, an electron transport layer having two or more layers is included between the light emitting layer and the second electrode. The electron transport layer having two or more layers may include materials which are the same as or different from each other.

According to an exemplary embodiment of the present specification, the first electrode is an anode or a cathode.

According to an exemplary embodiment of the present specification, the second electrode is a cathode or an anode.

According to an exemplary embodiment of the present specification, the organic light emitting device may be a normal type organic light emitting device in which an anode, an organic material layer having one or more layers, and a cathode are sequentially stacked on a substrate.

According to an exemplary embodiment of the present specification, the organic light emitting device may be an inverted type organic light emitting device in which a cathode, an organic material layer having one or more layers, and an anode are sequentially stacked on a substrate.

For example, the structure of the organic light emitting device according to an exemplary embodiment of the present specification is exemplified in FIGS. 1 and 2. FIGS. 1 and 2 exemplify an organic light emitting device, and the

organic light emitting device is not limited thereto.

FIG. 1 exemplifies a structure of an organic light emitting device in which a first electrode 2, a light emitting layer 4, and a second electrode 3 are sequentially stacked on a substrate 1. The compound of Chemical Formula 1 is included in the light emitting layer 4.

FIG. 2 exemplifies a structure of an organic light emitting device in which a substrate 1, a first electrode 2, a first hole injection layer 5, a second hole injection layer 6, a hole transport layer 7, an electron blocking layer 8, a light emitting layer 4, a first electron transport layer 9, a second electron transport layer 10, an electron injection layer 11, and a second electrode 3 are sequentially stacked. In the structure described above, the compound may be included in the light emitting layer 4.

The organic light emitting device of the present specification may be manufactured by the materials and methods known in the art, except that the light emitting layer includes the compound, that is, the compound represented by Chemical Formula 1.

When the organic light emitting device includes a plurality of organic material layers, the organic material layers may be formed of the same material or different materials.

For example, the organic light emitting device of the present specification may be manufactured by sequentially stacking a first electrode, an organic material layer, and a second electrode on a substrate. In this case, the organic light emitting device may be manufactured by depositing a metal or a metal oxide having conductivity, or an alloy thereof on a substrate to form an anode, forming an organic material layer including a hole injection layer, a hole transport layer, a light emitting layer, and an electron transport layer thereon, and then depositing a material, which may be used as a cathode, thereon, by using a physical vapor deposition (PVD) method such as sputtering or e-beam evaporation. In addition to the method described above, an organic light emitting device may be manufactured by sequentially depositing a second electrode material, an organic material layer, and a first electrode material on a substrate.

Further, the compound represented by Chemical Formula 1 may be formed as an organic material layer by not only a vacuum deposition method, but also a solution application method when an organic light emitting device is manufactured. Here, the solution application method means spin coating, dip coating, doctor blading, inkjet printing, screen printing, a spray method, roll coating, and the like, but is not limited thereto.

In addition to the method described above, an organic light emitting device may be made by sequentially depositing a second electrode material, an organic material layer, and a first electrode material on a substrate. However, the manufacturing method is not limited thereto.

As the first electrode material, materials having a high work function are usually preferred so as to facilitate the injection of holes into an organic material layer. Examples thereof include: a metal, such as vanadium, chromium, copper, zinc, and gold, or an alloy thereof; a metal oxide, such as zinc oxide, indium oxide, indium tin oxide (ITO), and indium zinc oxide (IZO); a combination of a metal and an oxide, such as ZnO:Al or $SnO_2$:Sb; a conductive polymer, such as poly(3-methylthiophene), poly[3,4-(ethylene-1,2-dioxy)thiophene] (PEDOT), polypyrrole, and polyaniline; and the like, but are not limited thereto.

As the second electrode material, materials having a low work function are usually preferred so as to facilitate the injection of electrons into an organic material layer. Examples thereof include: a metal, such as magnesium, calcium, sodium, potassium, titanium, indium, yttrium, lithium, gadolinium, aluminum, silver, tin, and lead, or an alloy thereof; a multi-layer structured material, such as LiF/Al or $LiO_2$/Al; and the like, but are not limited thereto.

The light emitting layer may include a host material and a dopant material. When an additional light emitting layer is included in addition to a light emitting layer including the compound represented by Chemical Formula 1 according to an exemplary embodiment of the present specification, examples of a host material include a fused and/or non-fused aromatic ring derivative, a hetero ring-containing compound, or the like. Specific examples of the fused aromatic ring derivative include anthracene derivatives, pyrene derivatives, naphthalene derivatives, pentacene derivatives, phenanthrene compounds, fluoranthene compounds, and the like, and specific examples of the hetero ring-containing compound include dibenzofuran derivatives, ladder-type furan compounds, pyrimidine derivatives, and the like, but the examples are not limited thereto.

Examples of the dopant material include an aromatic amine derivative, a styrylamine compound, a boron complex, a fluoranthene compound, a metal complex, and the like. Specifically, the aromatic amine derivative is a fused aromatic ring derivative having a substituted or unsubstituted arylamine group, and examples thereof include pyrene, anthracene, chrysene, periflanthene, and the like having an arylamine group. Further, the styrylamine compound is a compound in which a substituted or unsubstituted arylamine is substituted with at least one arylvinyl group, and one or two or more substituents selected from the group consisting of an aryl group, a silyl group, an alkyl group, a cycloalkyl group, and an arylamine group are substituted or unsubstituted. Specific examples thereof include styrylamine, styryldiamine, styryltriamine, styryltetramine, and the like, but are not limited thereto. Further, examples of the metal complex include an iridium complex, a platinum complex, and the like, but are not limited thereto.

The hole injection layer is a layer which accepts holes from an electrode. It is preferred that hole injection material has an ability to transport holes, and has an effect of accepting holes from an anode and an excellent hole injection effect for

a light emitting layer or a light emitting material. Further, the hole injection material is preferably a material which is excellent in ability to prevent excitons produced from a light emitting layer from moving to an electron injection layer or an electron injection material. In addition, the hole injection material is preferably a material which is excellent in ability to form a thin film. In addition, the highest occupied molecular orbital (HOMO) of the hole injection material is preferably a value between the work function of the anode material and the HOMO of the neighboring organic material layer. Specific examples of the hole injection material include: metal porphyrin, oligothiophene, and arylamine-based organic materials; hexanitrile hexaazatriphenylene-based organic materials; quinacridone-based organic materials; perylene-based organic materials; polythiophene-based conductive polymers such as anthraquinone and polyaniline; and the like, but are not limited thereto.

According to an exemplary embodiment of the present specification, the hole injection layer includes a compound represented by the following Chemical Formula HI-1, but is not limited thereto.

[Chemical Formula HI-1]

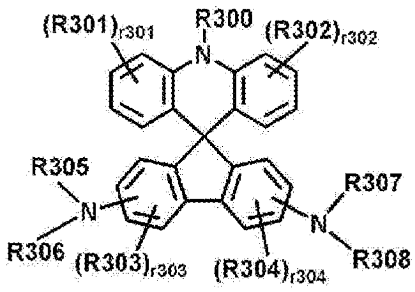

In Chemical Formula HI-1,

R300 to R308 are the same as or different from each other, and are each independently hydrogen; deuterium; a cyano group; a substituted or unsubstituted alkyl group; a substituted or unsubstituted amine group; a substituted or unsubstituted aryl group; or a substituted or unsubstituted heteroaryl group, or are bonded to an adjacent group to form a substituted or unsubstituted ring,
r301 and r302 are each an integer from 1 to 4,
r303 and r304 are each an integer from 1 to 3,
when r301 is 2 or higher, R301's are the same as or different from each other,
when r302 is 2 or higher, R302's are the same as or different from each other,
when r303 is 2 or higher, R303's are the same as or different from each other, and
when r304 is 2 or higher, R304's are the same as or different from each other.

According to an exemplary embodiment of the present specification, R301 to R304 are hydrogen.

According to an exemplary embodiment of the present specification, R300 is a substituted or unsubstituted aryl group.

According to an exemplary embodiment of the present specification, R300 is a monocyclic or polycyclic aryl group having 6 to 30 carbon atoms.

According to an exemplary embodiment of the present specification, R300 is a phenyl group.

According to an exemplary embodiment of the present specification, R300 is boned to R301 or R302 to form a substituted or unsubstituted ring.

According to an exemplary embodiment of the present specification, R300 is bonded to R301 or R302 to form an indole ring.

According to an exemplary embodiment of the present specification, R305 to R308 are the same as or different from each other, and are each independently a substituted or unsubstituted aryl group; or a substituted or unsubstituted heteroaryl group.

According to an exemplary embodiment of the present specification, R305 to R308 are the same as or different from each other, and are each independently a monocyclic or polycyclic aryl group having 6 to 30 carbon atoms; or a monocyclic or polycyclic heteroaryl group having 2 to 30 carbon atoms, which is unsubstituted or substituted with a monocyclic or polycyclic aryl group having 6 to 30 carbon atoms.

According to an exemplary embodiment of the present specification, R305 to R308 are the same as or different from each other, and are each independently a phenyl group; or a carbazole group unsubstituted or substituted with a phenyl group.

According to an exemplary embodiment of the present specification, Chemical Formula HI-1 is represented by

the following compound.

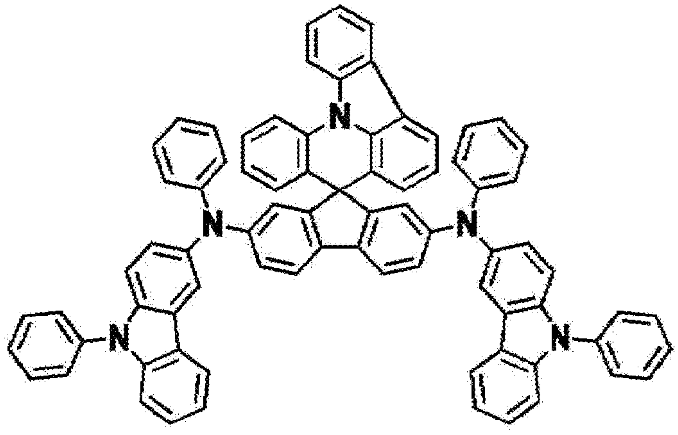

According to an exemplary embodiment of the present specification, the hole injection layer includes a compound represented by the following Chemical Formula HI-2, but is not limited thereto.

[Chemical Formula HI-2]

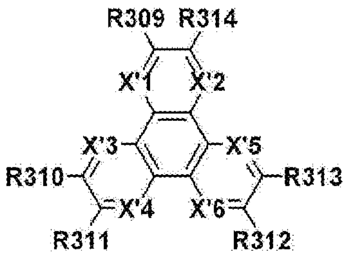

In Chemical Formula HI-2,

at least one of X'1 to X'6 is N, and the others are CH, and

R309 to R314 are the same as or different from each other, and are each independently hydrogen; deuterium; a cyano group; a substituted or unsubstituted alkyl group; a substituted or unsubstituted amine group; a substituted or unsubstituted aryl group; or a substituted or unsubstituted heteroaryl group, or are bonded to an adjacent group to form a substituted or unsubstituted ring.

According to an exemplary embodiment of the present specification, X'1 to X'6 are N.

According to an exemplary embodiment of the present specification, R309 to R314 are a cyano group.

According to an exemplary embodiment of the present specification, Chemical Formula HI-2 is represented by the following compound.

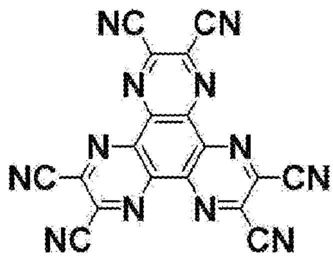

The hole transport layer is a layer which accepts holes from a hole injection layer and transports the holes to a light emitting layer. A hole transport material is preferably a material having high hole mobility which may accept holes from an anode or a hole injection layer and transfer the holes to a light emitting layer. Specific examples thereof include arylamine-based organic materials, conductive polymers, block copolymers having both conjugated portions and non-conjugated

portions, and the like, but are not limited thereto.

According to an exemplary embodiment of the present specification, the hole transport layer includes a compound represented by the following Chemical Formula HT-1, but is not limited thereto.

[Chemical Formula HT-1]

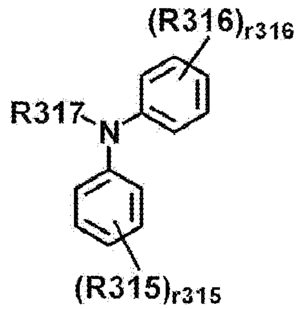

In Chemical Formula HT-1,

R315 to R317 are the same as or different from each other, and are each independently any one selected from the group consisting of hydrogen; deuterium; a substituted or unsubstituted alkyl group; a substituted or unsubstituted aryl group; a substituted or unsubstituted heteroaryl group; and a combination thereof, or are bonded to an adjacent group to form a substituted or unsubstituted ring,

r315 is an integer from 1 to 5, and when r315 is 2 or higher, two or more R315's are the same as or different from each other, and

r316 is an integer from 1 to 5, and when r316 is 2 or higher, two or more R316's are the same as or different from each other.

According to an exemplary embodiment of the present specification, R317 is any one selected from the group consisting of a substituted or unsubstituted aryl group; a substituted or unsubstituted heteroaryl group; and a combination thereof.

According to an exemplary embodiment of the present specification, R317 is any one selected from the group consisting of a carbazole group; a phenyl group; a biphenyl group; and a combination thereof.

According to an exemplary embodiment of the present specification, R315 and R316 are the same as or different from each other, and are each independently a substituted or unsubstituted aryl group, or are bonded to an adjacent group to form an aromatic hydrocarbon ring substituted with an alkyl group.

According to an exemplary embodiment of the present specification, R315 and R316 are the same as or different from each other, and are each independently a phenyl group, or are bonded to an adjacent group to form indene substituted with a methyl group.

According to an exemplary embodiment of the present specification, Chemical Formula HT-1 is represented by any one of the following compounds.

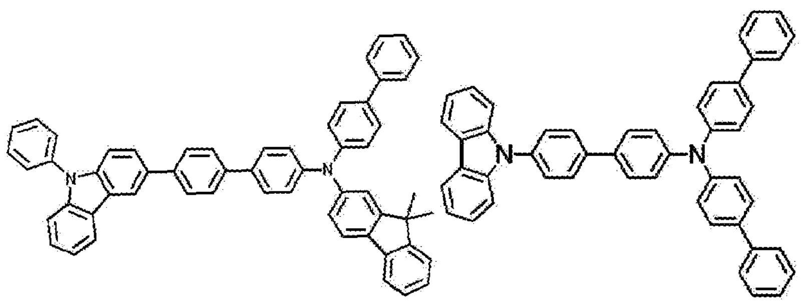

The electron transport layer is a layer which accepts electrons from an electron injection layer and transports the electrons to a light emitting layer. An electron transport material is preferably a material having high electron mobility which may proficiently accept electrons from a cathode and transfer the electrons to a light emitting layer. Specific examples thereof include: Al complexes of 8-hydroxyquinoline; complexes including $Alq_3$; organic radical compounds; hydroxy-flavone-metal complexes; and the like, but are not limited thereto. The electron transport layer may be used with any

desired cathode material, as used according to the related art. In particular, an appropriate cathode material is a typical material which has a low work function, followed by an aluminum layer or a silver layer. Specific examples thereof include cesium, barium, calcium, ytterbium, and samarium, in each case followed by an aluminum layer or a silver layer.

According to an exemplary embodiment of the present specification, the electron transport layer includes a compound represented by the following Chemical Formula ET-1.

[Chemical Formula ET-1]

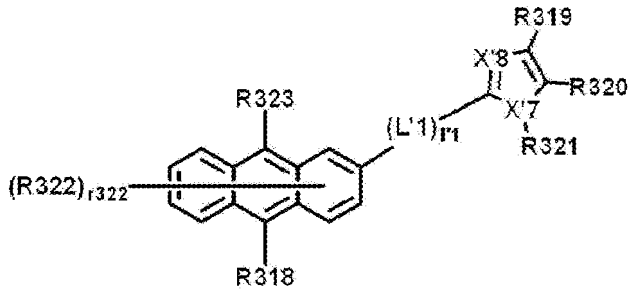

In Chemical Formula ET-1,

at least one of X'7 and X'8 is N, and the other is CH,
R318 to R323 are the same as or different from each other, and are each independently hydrogen; deuterium; a cyano group; a substituted or unsubstituted alkyl group; a substituted or unsubstituted amine group; a substituted or unsubstituted aryl group; or a substituted or unsubstituted heteroaryl group, or are bonded to an adjacent group to form a substituted or unsubstituted ring,
L'1 is a direct bond; a substituted or unsubstituted arylene group; or a substituted or unsubstituted heteroarylene group,
r322 is an integer from 1 to 7, and when r322 is 2 or higher, R322's are the same as or different from each other, and
l'1 is an integer from 1 to 5, and when l'1 is 2 or higher, L'1's are the same as or different from each other.

According to an exemplary embodiment of the present specification, R318 to R323 are the same as or different from each other, and are each independently hydrogen; or a substituted or unsubstituted aryl group, or adjacent groups are bonded to each other to form a substituted or unsubstituted aromatic hydrocarbon ring.

According to an exemplary embodiment of the present specification, R318 to R323 are the same as or different from each other, and are each independently hydrogen; a phenyl group; or a naphthyl group, or adjacent groups are bonded to each other to form a benzene.

According to an exemplary embodiment of the present specification, L'1 is a substituted or unsubstituted arylene group.

According to an exemplary embodiment of the present specification, L'1 is a phenylene group.

According to an exemplary embodiment of the present specification, ET-1 is represented by the following compound.

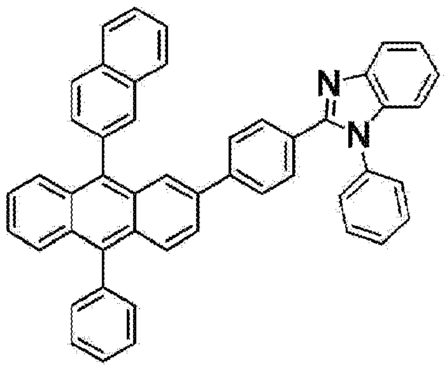

According to an exemplary embodiment of the present specification, the electron transport layer includes a compound represented by the following Chemical Formula ET-2.

[Chemical Formula ET-2]

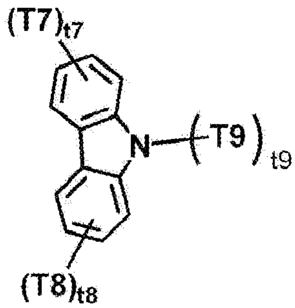

In Chemical Formula ET-2,

T7 to T9 are the same as or different from each other, and are each independently any one selected from the group consisting of hydrogen; deuterium; a cyano group; a substituted or unsubstituted alkyl group; a substituted or unsubstituted amine group; a substituted or unsubstituted aryl group; a substituted or unsubstituted heteroaryl group; and a combination thereof, or are bonded to an adjacent group to form a substituted or unsubstituted ring,
t7 is an integer from 1 to 4, and when t7 is 2 or higher, two or more T1's are the same as or different from each other,
t8 is an integer from 1 to 4, and when t8 is 2 or higher, two or more T2's are the same as or different from each other, and
t9 is an integer from 1 to 10, and when t9 is 2 or higher, two or more T3's are the same as or different from each other.

According to an exemplary embodiment of the present specification, T7 to T9 are the same as or different from each other, and are each independently any one selected from the group consisting of hydrogen; a substituted or unsubstituted aryl group; and a substituted or unsubstituted heteroaryl group, and a combination thereof.
According to an exemplary embodiment of the present specification, T7 to T9 are the same as or different from each other, and are each independently any one selected from the group consisting of hydrogen; a carbazole group; a phenyl group; a biphenyl group; a triazine group; and a combination thereof.
According to an exemplary embodiment of the present specification, ET-2 is represented by the following compound.

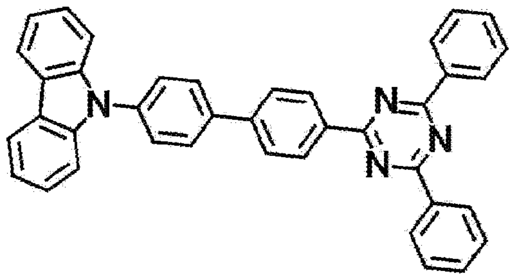

The electron transport layer may further include a metal complex. As the metal, a metal used in the art may be used.
The electron injection layer is a layer which accepts electrons from an electrode. It is preferred that an electron injection material is excellent in ability to transport electrons and has an effect of accepting electrons from the cathode and an excellent electron injection effect for a light emitting layer or a light emitting material. Further, the electron injection material is preferably a material which prevents excitons produced from a light emitting layer from moving to a hole injection layer and is excellent in ability to form a thin film. Specific examples thereof include fluorenone, anthraquinodimethane, diphenoquinone, thiopyran dioxide, oxazole, oxadiazole, triazole, imidazole, perylenetetracarboxylic acid, fluorenylidene methane, anthrone, and the like, and derivatives thereof, metal complex compounds, nitrogen-containing 5-membered ring derivatives, and the like, but are not limited thereto.
Examples of the metal complex compounds include 8-hydroxyquinolinato lithium, bis(8-hydroxyquinolinato) zinc, bis(8-hydroxyquinolinato) copper, bis(8-hydroxyquinolinato) manganese, tris(8-hydroxyquinolinato) aluminum, tris(2-methyl-8-hydroxyquinolinato) aluminum, tris(8-hydroxyquinolinato) gallium, bis(10-hydroxybenzo[h]quinolinato) beryllium, bis(10-hydroxybenzo[h]quinolinato) zinc, bis(2-methyl-8-quinolinato) chlorogallium, bis(2-methyl-8-quinolinato) (o-cresolato) gallium, bis(2-methyl-8-quinolinato) (1-naphtholato) aluminum, bis(2-methyl-8-quinolinato) (2-naphtholato) gallium, and the like, but are not limited thereto.

The electron blocking layer is a layer which may improve the service life and efficiency of a device by preventing electrons injected from an electron injection layer from passing through a light emitting layer and entering a hole injection layer. As the electron blocking layer, the publicly-known material can be used without limitation, and the electron blocking layer may be formed between a light emitting layer and a hole injection layer, between a light emitting layer and a hole transport layer, or between a light emitting layer and a layer which simultaneously injects and transports holes.

The hole blocking layer is a layer which blocks holes from reaching a cathode, and may be generally formed under the same conditions as those of the electron injection layer. Specific examples thereof include oxadiazole derivatives or triazole derivatives, phenanthroline derivatives, aluminum complexes, and the like, but are not limited thereto.

The organic light emitting device according to the present specification may be a top emission type, a bottom emission type, or a dual emission type according to the materials to be used.

The organic light emitting device according to the present specification may be included and used in various electronic devices. For example, the electronic device may be a display panel, a touch panel, a solar module, a lighting device, and the like, and is not limited thereto.

Hereinafter, the present specification will be described in detail with reference to Examples, Comparative Examples, and the like for specifically describing the present specification. However, the Examples and the Comparative Examples according to the present specification may be modified in various forms, and it is not interpreted that the scope of the present specification as set out in the appended claims is limited to the Examples and the Comparative Examples described below in detail. The Examples and the Comparative Examples of the present specification are provided to more completely explain the present specification to a person with ordinary skill in the art.

Synthesis Example 1. Synthesis of Core-1

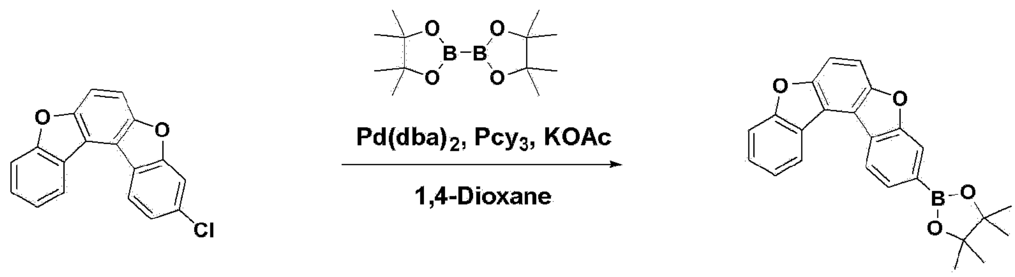


Core-1

<1-a> Preparation of Compound Core-1

3-Chloro-benzo[1,2-b:4,3-b']bisbenzofuran (50 g, 170.81 mmol), bis(pinacolato)diboron (54.3 g, 256.22 mmol), tricyclohexylphosphine (1.9 g, 6.7 mmol), potassium acetate (33.5 g, 341.6 mmol) and $Pd(dba)_2$ (1.9 g, 3.4 mmol) were added to 1,4-dioxane (400 ml), and the resulting solution was refluxed for 6 hours. The reaction solution was cooled, and the organic layer was extracted with ethyl acetate, and then dried over anhydrous magnesium sulfate. The organic solvent was removed under reduced pressure, and the residue was purified using column chromatography to obtain Compound Core-1 (44.0 g, yield 67%).

MS: [M+H]+ = 385

Synthesis Example 2. Synthesis of Core-2

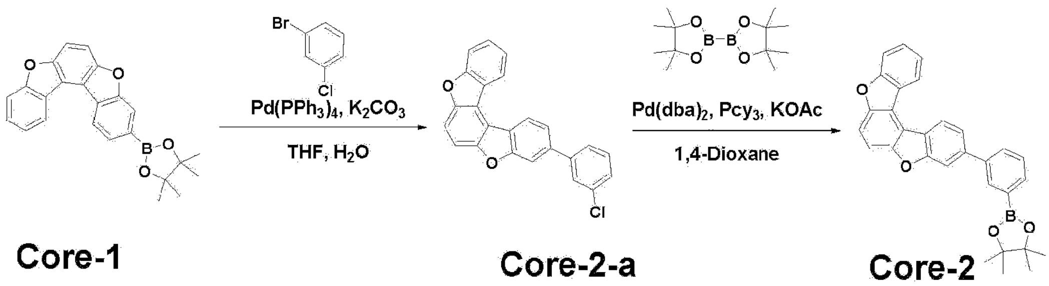


Core-1 Core-2-a Core-2

<2-A> Preparation of Compound Core-2-a

After Compound Core-1 (40 g, 104.1 mmol) and 1-bromo-3-chlorobenzene (18.9 g, 98.9 mmol) were dissolved in THF (400 ml), $Pd(PPh_3)_4$ (1.2 g, 1.0 mmol) and 80 ml of an aqueous 2M $K_2CO_3$ solution were added thereto, and the resulting solution was refluxed for 4 hours. The reaction solution was cooled, and the organic layer was extracted with ethyl acetate, and then dried over anhydrous magnesium sulfate. The organic solvent was removed under reduced pressure, and the residue was purified using column chromatography to obtain Compound Core-2-a (23.7 g, yield 65%) .
MS: [M+H]+ = 369

<2-b> Preparation of Compound Core-2

Compound Core-2 was obtained by performing synthesis and purification in the same manner as in <1-a> Preparation of Compound Core-1 of Synthesis Example 1, except that Compound Core-2-a was used instead of 3-chlorobenzo[1,2-b:4,3-b']bisbenzofuran in <1-a> Preparation of Compound Core-1 of Synthesis Example 1.
MS: [M+H]+ = 461

Synthesis Example 3. Synthesis of Core-3

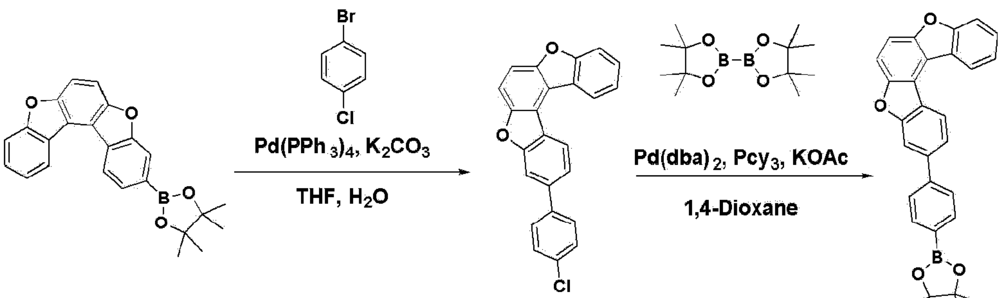


Core-1 Core-3-a Core-3

<3-a> Preparation of Compound Core-3-a

Compound Core-3-a was obtained by performing synthesis and purification in the same manner as in <2-a> Preparation of Compound Core-2-a of Synthesis Example 2, except that 1-bromo-4-chlorobenzene was used instead of 1-bromo-3-chlorobenzene in <2-a> Preparation of Compound Core-2-a of Synthesis Example 2.
MS: [M+H]+ = 369

<3-b> Preparation of Compound Core-3

Compound Core-3 was obtained by performing synthesis and purification in the same manner as in <1-a> Preparation of Compound Core-1 of Synthesis Example 1, except that Compound Core-3-a was used instead of 3-chlorobenzo[1,2-b:4,3-b']bisbenzofuran in <1-a> Preparation of Compound Core-1 of Synthesis Example 1.
MS: [M+H]+ = 461

Synthesis Example 4. Synthesis of Core-4

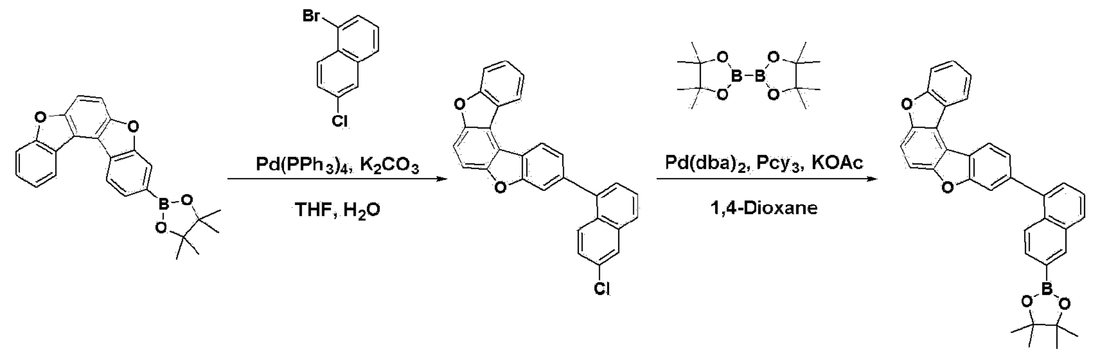


Core-1 Core-4-a Core-4

<4-a> Preparation of Compound Core-4-a

Compound Core-4-a was obtained by performing synthesis and purification in the same manner as in <2-a> Preparation of Compound Core-2-a of Synthesis Example 2, except that 1-bromo-6-chloronaphthalene was used instead of 1-bromo-3-chlorobenzene in <2-a> Preparation of Compound Core-2-a of Synthesis Example 2.
MS: [M+H]+ = 419

<4-b> Preparation of Compound Core-4

Compound Core-4 was obtained by performing synthesis and purification in the same manner as in <1-a> Preparation of Compound Core-1 of Synthesis Example 1, except that Compound Core-4-a was used instead of 3-chloro-benzo[1,2-b:4,3-b']bisbenzofuran in <1-a> Preparation of Compound Core-1 of Synthesis Example 1.
MS: [M+H]+ = 511

Synthesis Example 5. Synthesis of BH-1

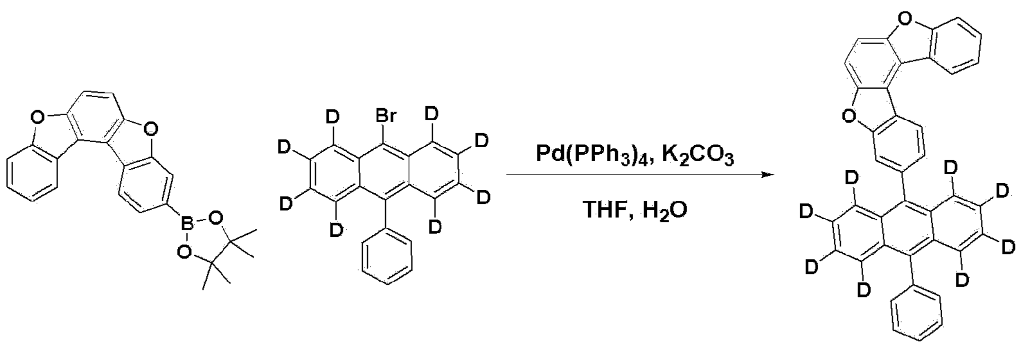


Core-1 BH-1

<5-a> Preparation of Compound BH-1

Compound BH-1 was obtained by performing synthesis and purification in the same manner as in <2-a> Preparation of Compound Core-2-a of Synthesis Example 2, except that 9-bromo-10-phenylanthracene-1,2,3,4,5,6,7,8-$d_8$ was used instead of 1-bromo-3-chlorobenzne in <2-a> Preparation of Compound Core-2-a of Synthesis Example 2.
MS: [M+H]+ = 519

Synthesis Example 6. Synthesis of BH-2

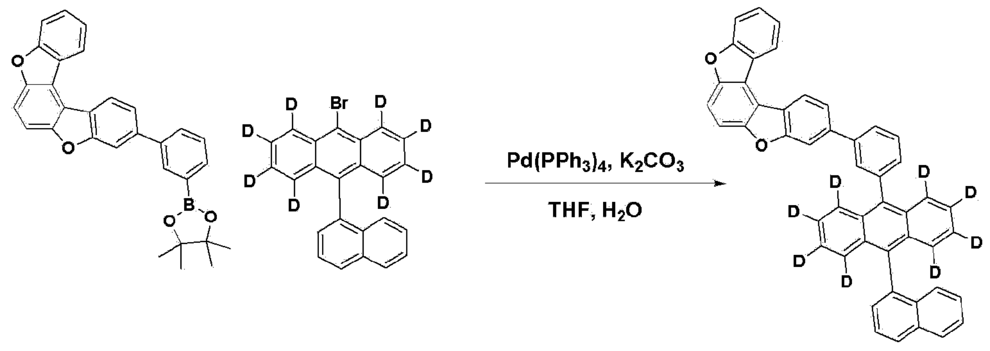


Core-2 BH-2

<6-a> Preparation of Compound BH-2

Compound BH-2 was obtained by performing synthesis and purification in the same manner as in <2-a> Preparation of Compound Core-2-a of Synthesis Example 2, except that 9-bromo-10-(naphthalen-1-yl)anthracene-1,2,3,4,5,6,7,8-$d_8$ was used instead of 1-bromo-3-chlorobenzene, and Compound Core-2 was used instead of Compound Core-1 in <2-a> Preparation of Compound Core-2-a of Synthesis Example 2.
MS: [M+H]+ = 645

Synthesis Example 7. Synthesis of BH-3

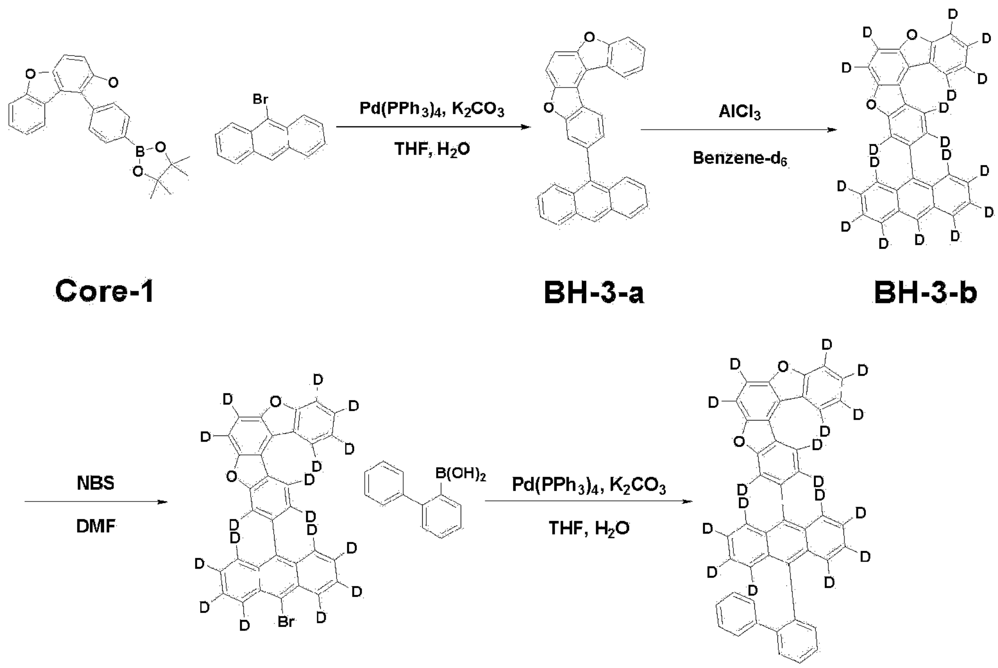


Core-1 BH-3-a BH-3-b

BH-3-c BH-3

<7-a> Preparation of Compound BH-3-a

Compound BH-3-a was obtained by performing synthesis and purification in the same manner as in <2-a> Preparation of Compound Core-2-a of Synthesis Example 2, except that 9-bromoanthracene was used instead of 1-bromo-3-chlorobenzene in <2-a> Preparation of Compound Core-2-a of Synthesis Example 2.
MS: [M+H]+ = 435

<7-b> Preparation of Compound BH-3-b

Compound BH-3-a (40 g, 92.1mmol) and $AlCl_3$ (8 g, 60 mmol) were added to $C_6D_6$ (800 ml), and the resulting solution was stirred for 5 hours. After the reaction was completed, $D_2O$ (50ml) was added thereto, the resulting solution was stirred for 30 minutes, and then trimethylamine (5ml) was added dropwise thereto. The reaction solution was transferred to a separatory funnel, and an extraction with water and toluene was performed. The extract was dried over $MgSO_4$, and then recrystallized with ethyl acetate to obtain BH-3-b (27.9 g, yield 67%).
MS: [M+H]+ = 453

<7-c> Preparation of Compound BH-3-c

After Compound BH-3-b (30 g, 66.3 mmol) was dispersed in 250 ml of dimethylformamide, a solution of n-bromosuccinimide (12.4 g, 69.6 mmol) dissolved in 50 ml of dimethylformamide was slowly added dropwise thereto. After reaction at room temperature for 4 hours, 600 ml of water was added dropwise thereto. When a solid was produced, the solid was filtered, and then dissolved in ethyl acetate, and the resulting solution was put into a separatory funnel, and then washed several times with distilled water. The resulting product was recrystallized in EA to obtain Compound BH-3-c (29.9 g, yield 85%).
MS: [M+H]+ = 531

<7-d> Preparation of Compound BH-3

Compound BH-3 was obtained by performing synthesis and purification in the same manner as in <2-a> Preparation of Compound Core-2-a of Synthesis Example 2, except that Compound BH-3-c was used instead of 1-bromo-3-chlorobenzene and [1,1'-biphenyl]-2-ylboronic acid was used instead of Compound Core-1 in <2-a> Preparation of Compound Core-2-a of Synthesis Example 2.
MS: [M+H]+ = 604

Synthesis Example 8. Synthesis of BH-4

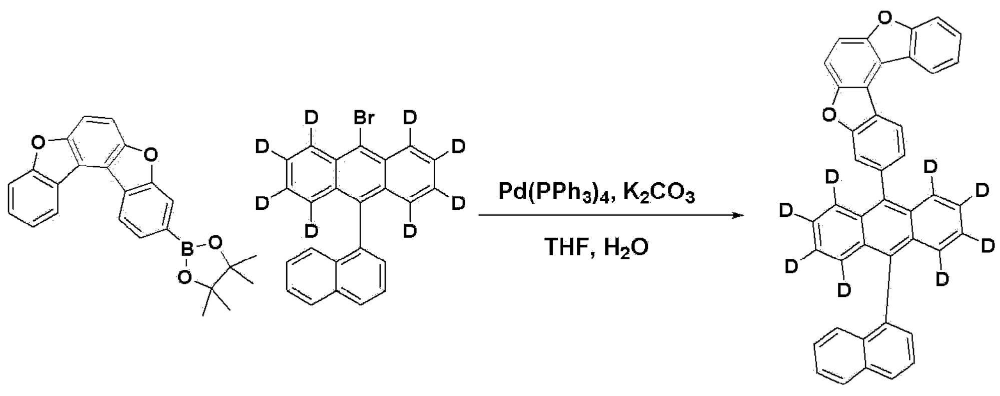


Core-1

BH-4

<8-a> Preparation of Compound BH-4

Compound BH-4 was obtained by performing synthesis and purification in the same manner as in <2-a> Preparation of Compound Core-2-a of Synthesis Example 2, except that 9-bromo-10-(naphthalen-1-yl)anthracene-1,2,3,4,5,6,7,8-$d_8$ was used instead of 1-bromo-3-chlorobenzene in <2-a> Preparation of Compound Core-2-a of Synthesis Example 2.
MS: [M+H]+ = 569

Synthesis Example 9. Synthesis of BH-5

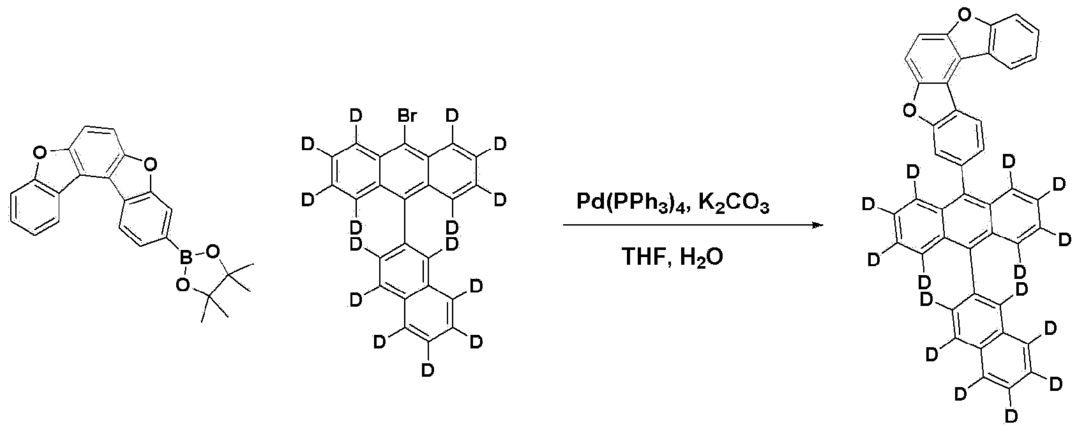


Core-1 BH-5

<9-a> Preparation of Compound BH-5

Compound BH-5 was obtained by performing synthesis and purification in the same manner as in <2-a> Preparation of Compound Core-2-a of Synthesis Example 2, except that 9-bromo-10-(naphthalen-2-yl-d7)anthracene-1,2,3,4,5,6,7,8-$d_8$ was used instead of 1-bromo-3-chlorobenzene in <2-a> Preparation of Compound Core-2-a of Synthesis Example 2.
MS: [M+H]+ = 576

Synthesis Example 10. Synthesis of BH-6

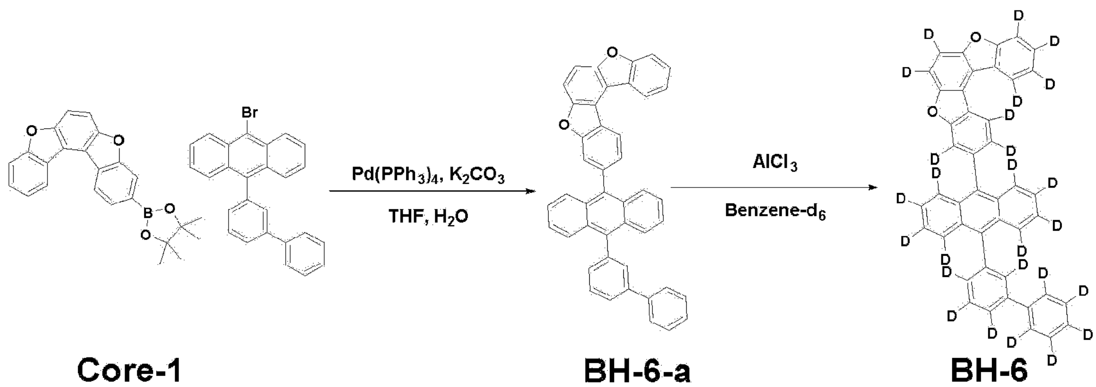


Core-1 BH-6-a BH-6

<10-a> Preparation of Compound BH-6-a

Compound BH-6-a was obtained by performing synthesis and purification in the same manner as in <2-a> Preparation of Compound Core-2-a of Synthesis Example 2, except that 9-([1,1'-biphenyl]-3-yl)-10-bromoanthracene was used instead of 1-bromo-3-chlorobenzene in <2-a> Preparation of Compound Core-2-a of Synthesis Example 2.
MS: [M+H]+ = 587

<10-b> Preparation of Compound BH-6

Compound BH-6 was obtained by performing synthesis and purification in the same manner as in <7-b> Preparation of Compound BH-3-b of Synthesis Example 7, except that Compound BH-6-a was used instead of Compound

BH-3-a in <7-b> Preparation of Compound BH-3-b of Synthesis Example 7.
MS: [M+H]+ = 613

Synthesis Example 11. Synthesis of BH-7

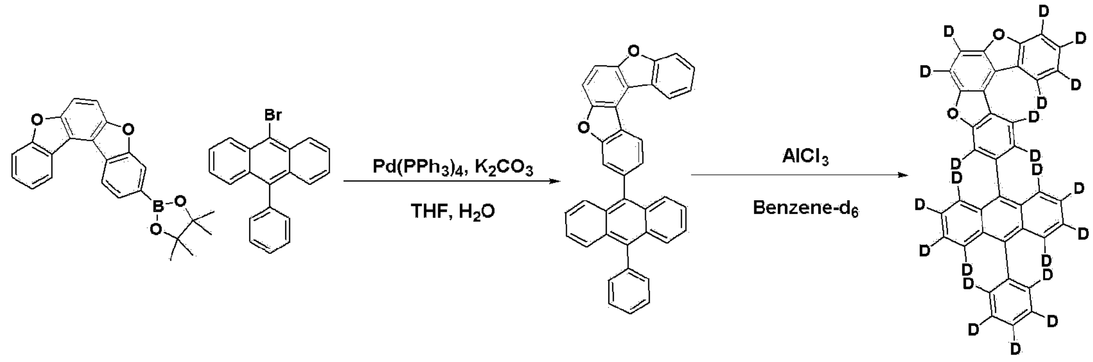


Core-1 BH-7-a BH-7

<11-a> Preparation of Compound BH-7-a

Compound BH-7-a was obtained by performing synthesis and purification in the same manner as in <2-a> Preparation of Compound Core-2-a of Synthesis Example 2, except that 9-bromo-10-phenylanthracene was used instead of 1-bromo-3-chlorobenzene in <2-a> Preparation of Compound Core-2-a of Synthesis Example 2.
MS: [M+H]+ = 511

<11-b> Preparation of Compound BH-7

Compound BH-7 was obtained by performing synthesis and purification in the same manner as in <7-b> Preparation of Compound BH-3-b of Synthesis Example 7, except that Compound BH-7-a was used instead of Compound BH-3-a in <7-b> Preparation of Compound BH-3-b of Synthesis Example 7.
MS: [M+H]+ = 533

Synthesis Example 12. Synthesis of BH-8

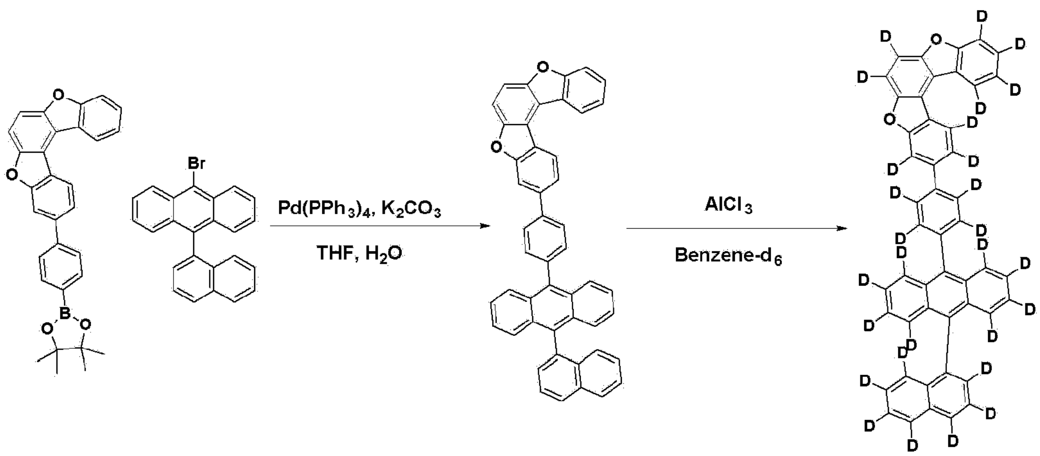


Core-3 BH-8-a BH-8

<12-a> Preparation of Compound BH-8-a

Compound BH-8-a was obtained by performing synthesis and purification in the same manner as in <2-a> Preparation of Compound Core-2-a of Synthesis Example 2, except that Compound Core-3 was used instead of Compound Core-1 and 9-bromo-10-(naphthalen-1-yl)anthracene was used instead of 1-bromo-3-chlorobenzene in <2-a> Preparation of Compound Core-2-a of Synthesis Example 2.
MS: [M+H]+ = 637

<12-b> Preparation of Compound BH-8

Compound BH-8 was obtained by performing synthesis and purification in the same manner as in <7-b> Preparation of Compound BH-3-b of Synthesis Example 7, except that Compound BH-8-a was used instead of Compound BH-3-a.
MS: [M+H]+ = 665

Synthesis Example 13. Synthesis of BH-9

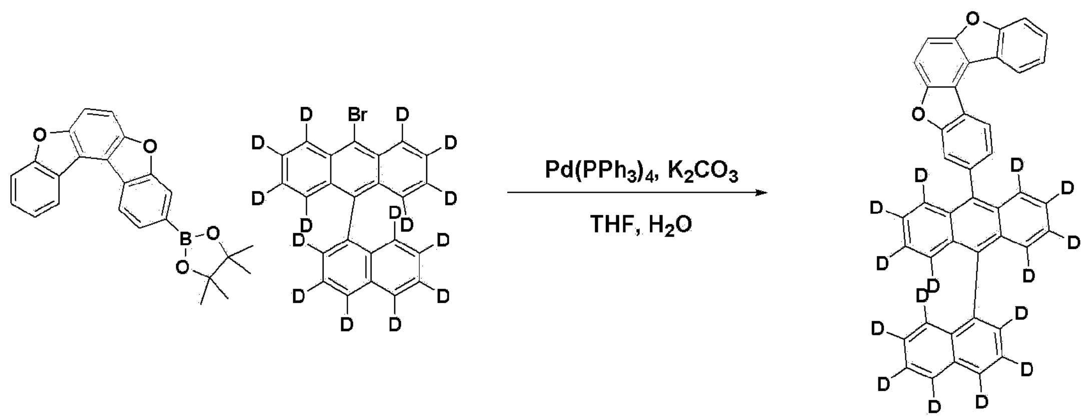


Core-1

BH-9

<13-a> Preparation of Compound BH-9

Compound BH-9 was obtained by performing synthesis and purification in the same manner as in <2-a> Preparation of Compound Core-2-a of Synthesis Example 2, except that 9-bromo-10-(naphthalen-1-yl-d7)anthracene-1,2,3,4,5,6,7,8-$d_8$ was used instead of 1-bromo-3-chlorobenzene in <2-a> Preparation of Compound Core-2-a of Synthesis Example 2.
MS: [M+H]+ = 576

Synthesis Example 14. Synthesis of BH-10

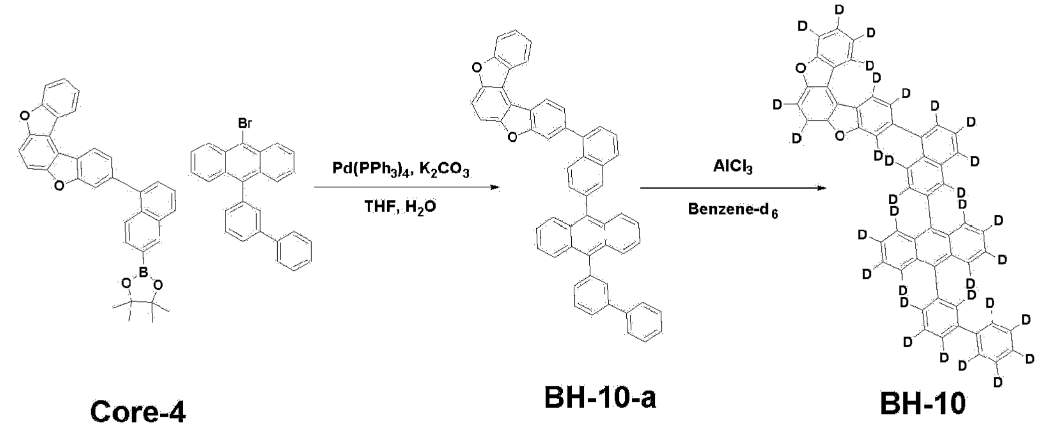


Core-4 BH-10-a BH-10

<14-a> Preparation of Compound BH-10-a

Compound BH-10-a was obtained by performing synthesis and purification in the same manner as in <2-a> Preparation of Compound Core-2-a of Synthesis Example 2, except that Compound Core-4 was used instead of Compound Core-1 and 9-([1,1'-biphenyl]-3-yl)-10-bromoanthracene was used instead of 1-bromo-3-chlorobenzene in <2-a> Preparation of Compound Core-2-a of Synthesis Example 2.
MS: [M+H]+ = 713

<14-b> Preparation of Compound BH-10

Compound BH-10 was obtained by performing synthesis and purification in the same manner as in <7-b> Preparation of Compound BH-3-b of Synthesis Example 7, except that Compound BH-10-a was used instead of Compound BH-3-a.
MS: [M+H]+ = 746

Synthesis Example 15. Synthesis of BH-11

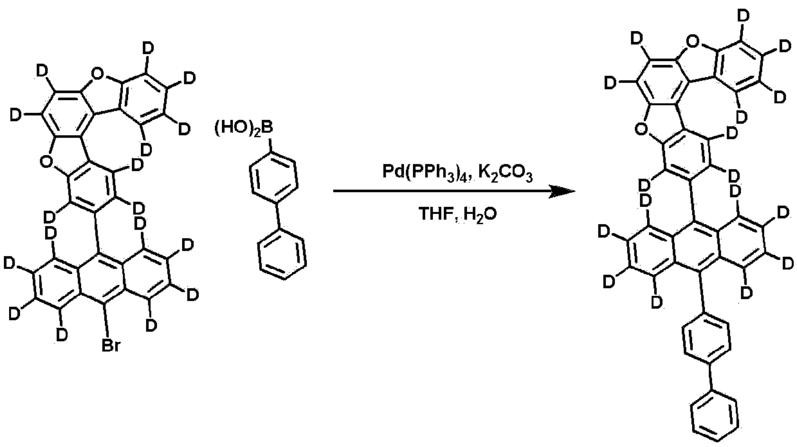


BH-3-c BH-11

<15-a> Preparation of Compound BH-11

Compound BH-11 was obtained by performing synthesis and purification in the same manner as in <2-a> Preparation of Compound Core-2-a of Synthesis Example 2, except that Compound BH-3-c was used instead of 1-bromo-3-chlorobenzene and [1,1'-biphenyl]-4-ylboronic acid was used instead of Compound Core-1 in <2-a> Preparation of Compound Core-2-a of Synthesis Example 2.
MS: [M+H]+ = 604

Synthesis Example 16. Synthesis of BH-12

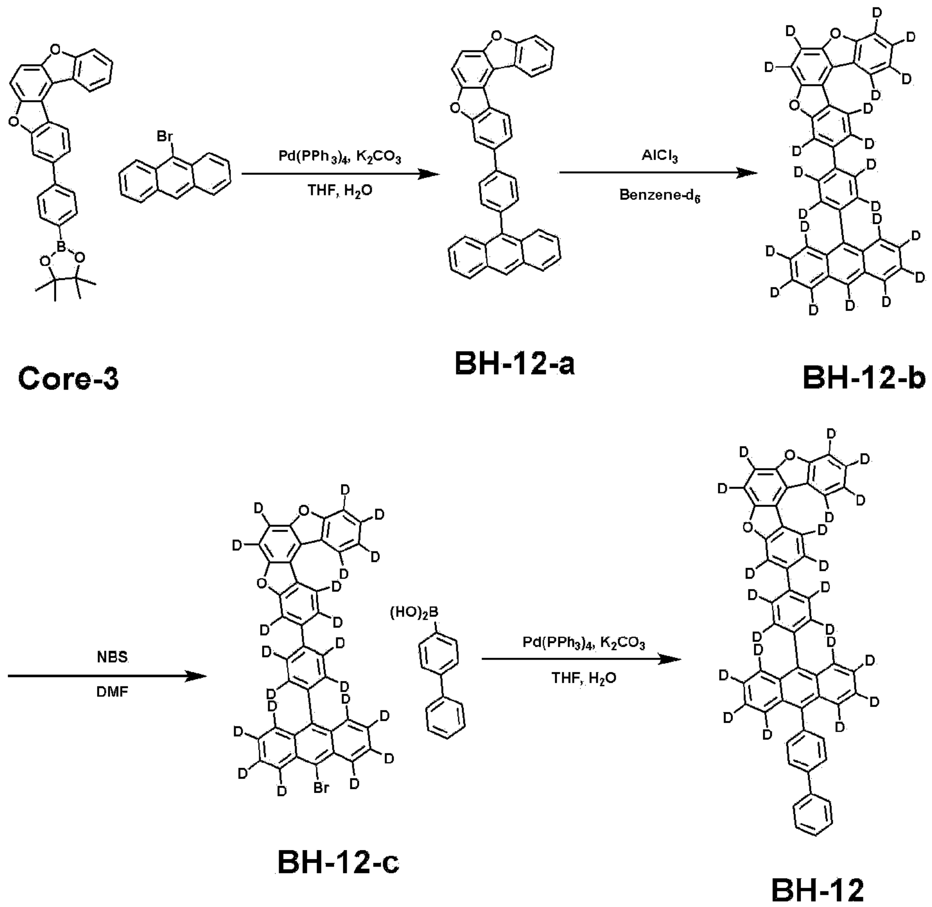


Core-3 BH-12-a BH-12-b

BH-12-c BH-12

<16-a> Preparation of Compound BH-12-a

Compound BH-12-a was obtained by performing synthesis and purification in the same manner as in <2-a> Preparation of Compound Core-2-a of Synthesis Example 2, except that 9-bromoanthracene was used instead of 1-bromo-3-chlorobenzene and Compound Core-3 was used instead of Compound Core-1 in <2-a> Preparation of Compound Core-2-a of Synthesis Example 2.
MS: [M+H]+ = 511

<16-b> Preparation of Compound BH-12-b

Compound BH-12-b was obtained by performing synthesis and purification in the same manner as in <7-b> Preparation of Compound BH-3-b of Synthesis Example 7, except that Compound BH-12-a was used instead of Compound BH-3-a in <7-b> Preparation of Compound BH-3-b of Synthesis Example 7.
MS: [M+H]+ = 533

<16-c> Preparation of Compound BH-12-c

Compound BH-12-c was obtained by performing synthesis and purification in the same manner as in <7-c> Preparation of Compound BH-3-b of Synthesis Example 7, except that Compound BH-12-b was used instead of Compound BH-3-a in <7-c> Preparation of Compound BH-3-c of Synthesis Example 7.
MS: [M+H]+ = 611

<16-d> Preparation of Compound BH-12

Compound BH-12 was obtained by performing synthesis and purification in the same manner as in <2-a> Preparation of Compound Core-2-a of Synthesis Example 2, except that Compound BH-12-c and [1,1'-biphenyl]-4-ylboronic acid were used instead of 1-bromo-3-chlorobenzene and Compound Core-1, respectively.
MS: [M+H]+ = 684

<Experimental Example 1: Manufacture of Organic Light Emitting Device>

In the following Examples 1-9 and 11-12 are Reference Examples not according to the invention.

Reference Example 1.

A glass substrate thinly coated with indium tin oxide (ITO) to have a thickness of 1,400 Å was put into distilled water in which a detergent was dissolved, and ultrasonically washed. In this case, a product manufactured by Fischer Co., was used as the detergent, and distilled water, which had been filtered twice with a filter manufactured by Millipore Co., was used as the distilled water. After the ITO was washed for 30 minutes, ultrasonic washing was conducted twice repeatedly using distilled water for 10 minutes. After the washing using distilled water was completed, ultrasonic washing was conducted by using isopropyl alcohol, acetone, and methanol solvents, and the resulting product was dried and then transported to a plasma washing machine. Furthermore, the substrate was cleaned by using oxygen plasma for 5 minutes, and then was transported to a vacuum deposition machine.

The following HI-A and HATCN were thermally vacuum deposited to have a thickness of 650 Å and 50 Å, respectively, on the ITO transparent electrode prepared as described, thereby sequentially forming first and second hole injection layers. The following HT-A was vacuum deposited to have a thickness of 600 Å on the second hole injection layer, thereby forming a hole transport layer. The following HT-B was vacuum deposited to have a thickness of 50 Å on the hole transport layer, thereby forming an electron blocking layer.

Subsequently, the following compound BD-A as a blue light emitting dopant was vacuum deposited at 4 wt% based on a total weight of the light emitting layer and the following BH-1 as a host was vacuum deposited at 96 wt% based on a total weight of the light emitting layer to a thickness of 200 Å on the electron blocking layer, thereby forming a light emitting layer.

Next, the following compound ET-A as a first electron transport layer was vacuum deposited to have a thickness of 50 Å on the light emitting layer, and subsequently, the following ET-B and LiQ were vacuum deposited at a weight ratio of 1:1, thereby forming a second electron transport layer having a thickness of 360 Å. LiQ was vacuum deposited to have a thickness of 5 Å on the second electron transport layer, thereby forming an electron injection layer. Aluminum and silver were deposited at a weight ratio of 10:1 to have a thickness of 220 Å on the electron injection layer, and aluminum was deposited to have a thickness of 1,000 Å thereon, thereby forming a negative electrode.

In the aforementioned procedure, the deposition rate of the organic materials was maintained at 0.4 to 0.9 Å/sec, the deposition rate of aluminum of the negative electrode was maintained at 2 Å/sec, and the degree of vacuum during the deposition was maintained at $1\times10^{-7}$ to $5\times10^{-8}$ torr, thereby manufacturing an organic light emitting device.

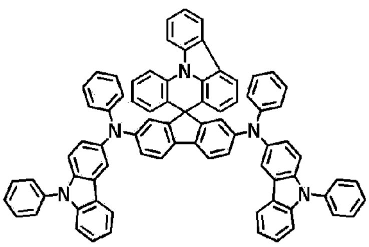

HI-A

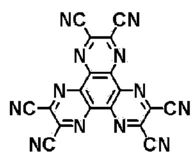

HATCN

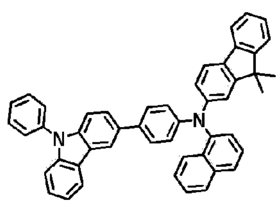

HT-A

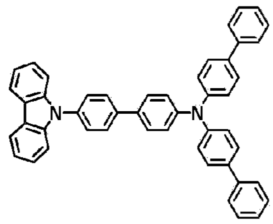
HT-B

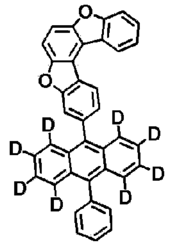
BH-1

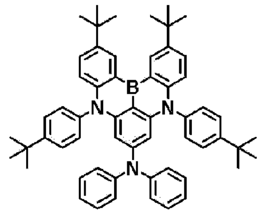
BD-A

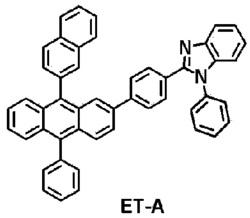
ET-A

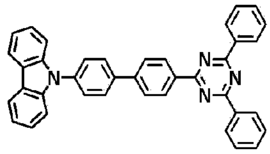
ET-B

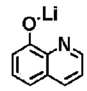
LiQ

Reference Examples 2 to 9, 11 to 12 and Example 10

Organic light emitting devices of Reference Examples 2 to 9, 11 to 12 and Example 10 were manufactured in the same manner as in Reference Example 1, except that compounds described in the following Table 1 were used as hosts of the light emitting layer instead of Compound BH-1 in Reference Example 1.

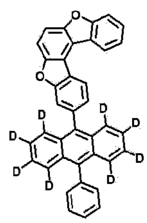
BH-1

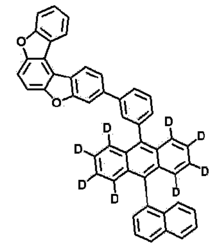
BH-2

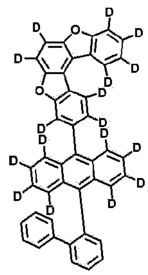
BH-3

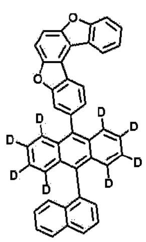
BH-4

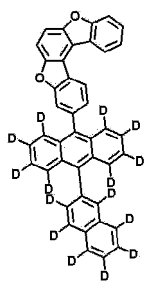
BH-5

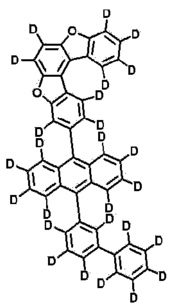
BH-6

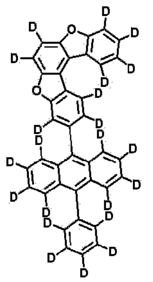
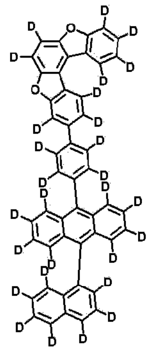
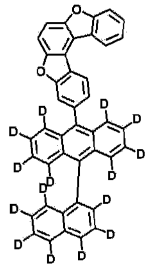

BH-7 BH-8 BH-9

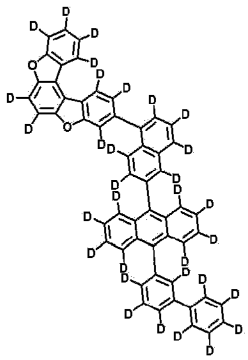
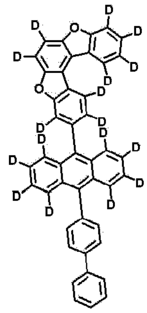
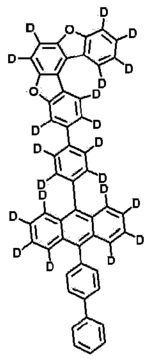

BH-10 BH-11 BH-12

Comparative Examples 1 to 5

Organic light emitting devices of Comparative Examples 1 to 5 were manufactured in the same manner as in Reference Example 1, except that the following Compounds BH-a to BH-e were used as hosts of the light emitting layer instead of Compound BH-1 in Reference Example 1.

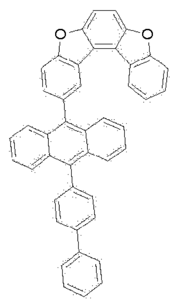
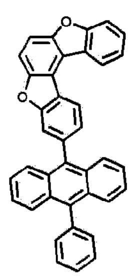
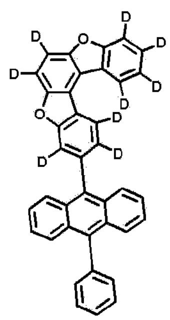
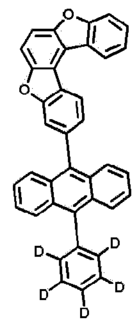
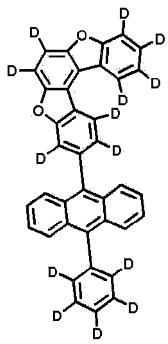

BH-a BH-b BH-c BH-d BH-e

Voltages and conversion efficiencies (cd/A/y) when a current density of 10 mA/cm2 was applied to the organic light emitting devices in Reference Examples 1 to 9, 11 to 12, Example 10 and Comparative Examples 1 to 5 and service lives ($LT_{95}$) when a current density of 20 mA/$cm^2$ was applied to the devices were measured, and the results are shown in the following Table 1. In this case, $LT_{95}$ denotes the ratio of time taken for the luminance to decrease to 95% when the initial luminance at the current density of 20 mA/$cm^2$ is set to 100%.

[Table 1]

| | Light emitting layer | 10 mA/cm$^2$ | | 20 mA/ cm$^2$ |
|---|---|---|---|---|
| | Host | Driving voltage (V) | Conversion efficiency (cd/A/y) | $LT_{95}$ (%) |
| Reference Example 1 | BH-1 | 3.70 | 43.7 | 143 |
| Reference Example 2 | BH-2 | 3.69 | 44.1 | 125 |
| Reference Example 3 | BH-3 | 3.67 | 44.7 | 161 |
| Reference Example 4 | BH-4 | 3.51 | 44.9 | 149 |
| Reference Example 5 | BH-5 | 3.67 | 44.3 | 132 |
| Reference Example 6 | BH-6 | 3.66 | 43.7 | 151 |
| Reference Example 7 | BH-7 | 3.69 | 43.9 | 161 |
| Reference Example 8 | BH-8 | 3.70 | 45.2 | 147 |
| Reference Example 9 | BH-9 | 3.52 | 45.0 | 155 |
| Example 10 | BH-10 | 3.63 | 45.1 | 139 |
| Reference Example 11 | BH-11 | 3.62 | 44.8 | 140 |
| Reference Example 12 | BH-12 | 3.69 | 45.1 | 138 |
| Comparative Example 1 | BH-a | 3.79 | 40.1 | 101 |
| Comparative Example 2 | BH-b | 3.71 | 43.1 | 97 |
| Comparative Example 3 | BH-c | 3.72 | 43.3 | 110 |
| Comparative Example 4 | BH-d | 3.71 | 43.5 | 105 |
| Comparative Example 5 | BH-e | 3.72 | 43.3 | 115 |

The conversion efficiency (cd/A/y) takes a current efficiency (cd/A) to color purity (CIEy) of the material into consideration, and is an important reference value for efficiency in small and large organic light emitting devices targeting high luminance and high color gamut.

In Table 1, Examples 1 to 9, 11 to 12 and Example 10, which are organic light emitting devices including a compound including at least one deuterium in anthracene, Comparative Examples 1 and 2 include a compound which does not include deuterium, and Comparative Examples 3 to 5 include a compound which does not include deuterium in the anthracene core.

Specifically, since the compound that always includes deuterium in the anthracene has structural characteristics that the mobility of electrons and holes is improved and molecular stability is improved compared to Comparative Examples 1 and 2 which do not include deuterium and Comparative Examples 3 to 5 which do not include deuterium in the anthracene core, it could be seen that an organic light emitting device including the compound that always includes deuterium in the anthracene is excellent in terms of driving voltage, efficiency and service life.

## Claims

**1.** A compound represented by the following Chemical Formula 1:

[Chemical Formula 1]

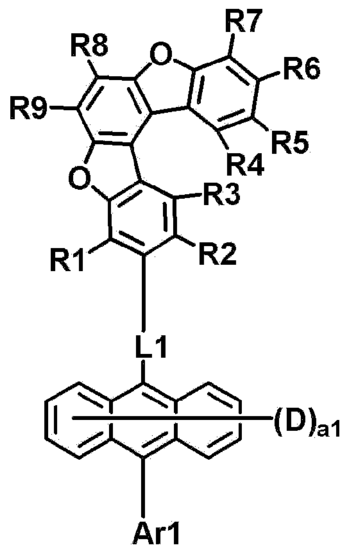

wherein, in Chemical Formula 1,
D is deuterium,
L1 is a naphthylene group unsubstituted or substituted with deuterium,
Ar1 is a substituted or unsubstituted aryl group,
R1 to R9 are the same as or different from each other, and are each independently hydrogen; or deuterium, and
a1 is an integer from 1 to 8.

2. The compound of claim 1, wherein Chemical Formula 1 is represented by the following Chemical Formula 1-2:

[Chemical Formula 1-2]

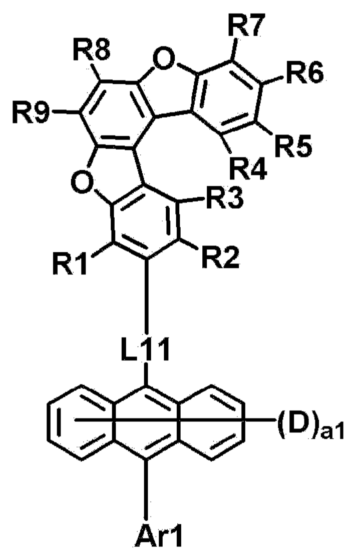

in Chemical Formula 1-2,

the definitions of D, Ar1, a1 and R1 to R9 are the same as those defined in Chemical Formula 1, and
L11 is a naphthylene group unsubstituted or substituted with deuterium.

3. The compound of claim 1, wherein Chemical Formula 1 is represented by the following Chemical Formula 1-4:

[Chemical Formula 1-4]

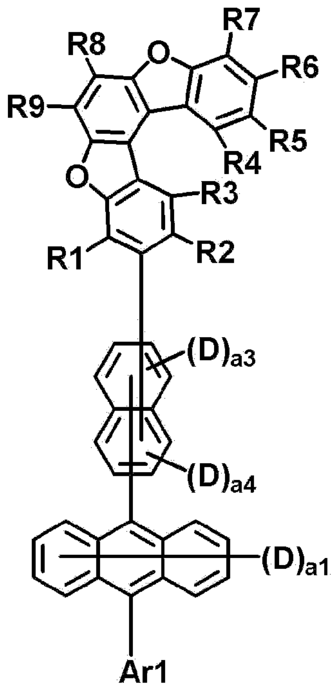

in Chemical Formula 1-4,

the definitions of D, Ar1, a1 and R1 to R9 are the same as those defined in Chemical Formula 1,
a3 is an integer from 0 to 4,
a4 is an integer from 0 to 4, and

$$0 \leq a3 + a4 \leq 6.$$

4. The compound of claim 1, wherein Ar1 is a monocyclic or polycyclic aryl group having 6 to 30 carbon atoms, which is unsubstituted or substituted with one or more substituents of deuterium, a monocyclic or polycyclic aryl group having 6 to 30 carbon atoms, or a combination thereof.

5. The compound of claim 1, wherein Chemical Formula 1 is any one selected from the following compounds:

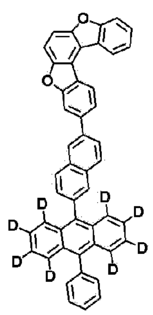

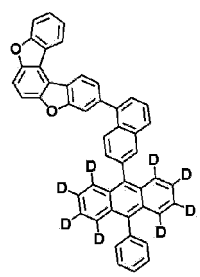

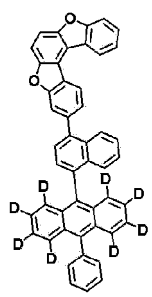

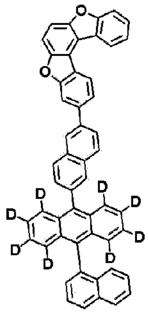
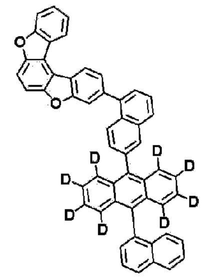
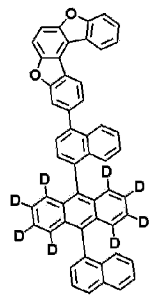

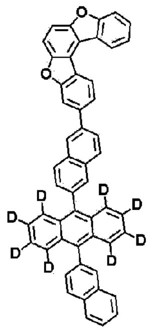
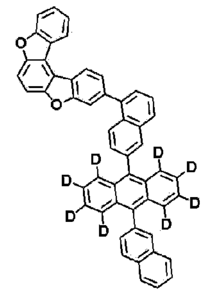
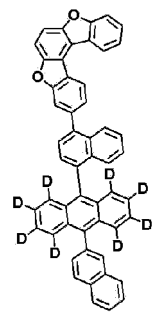

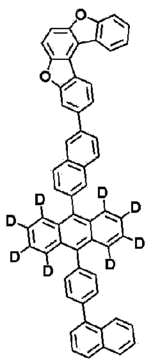
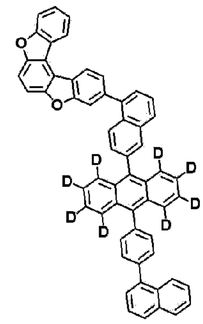
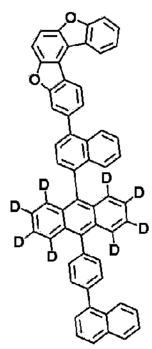

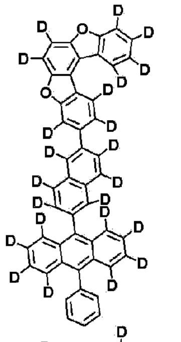

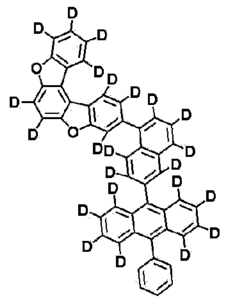

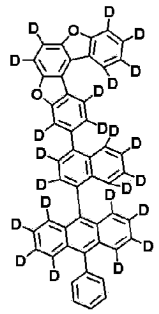

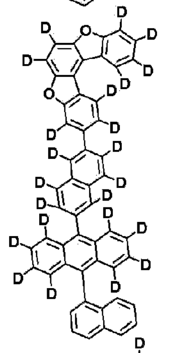

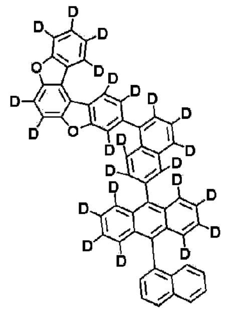

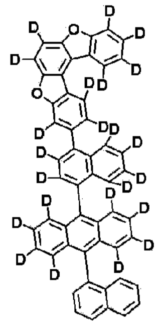

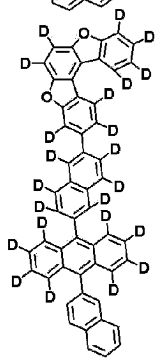

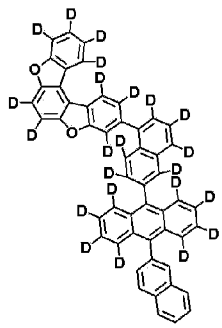

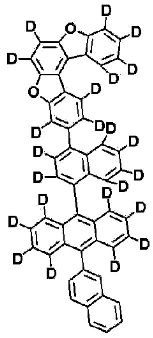

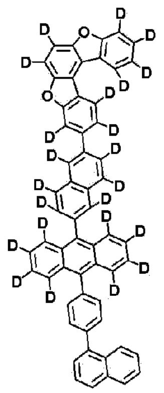

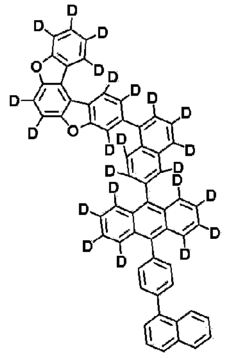

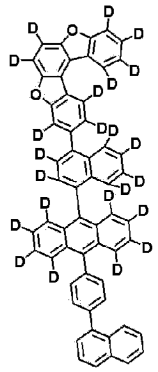

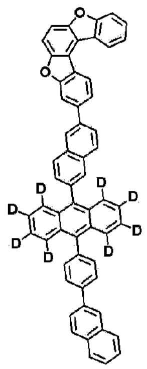
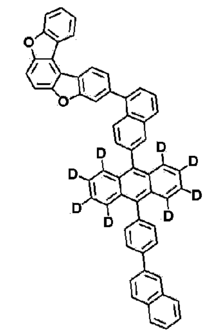
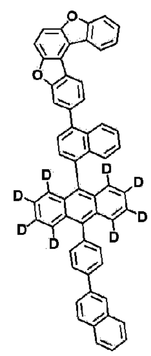

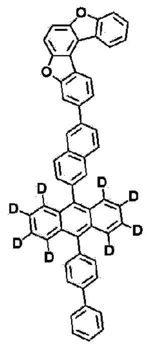
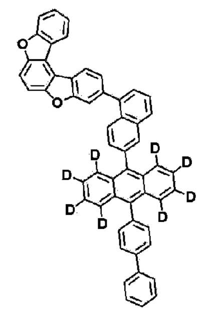
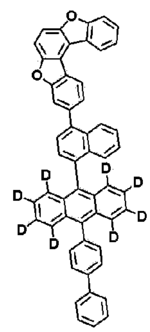

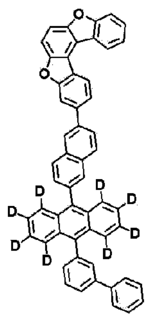
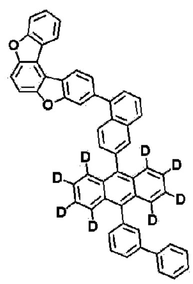
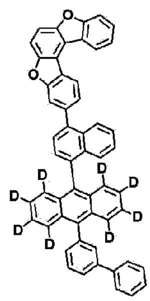

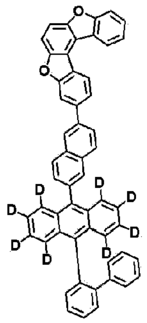
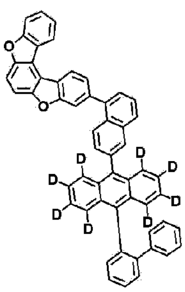
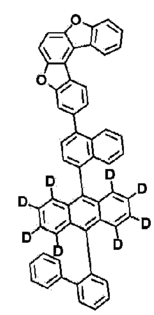

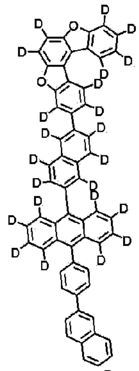
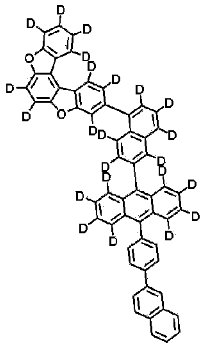
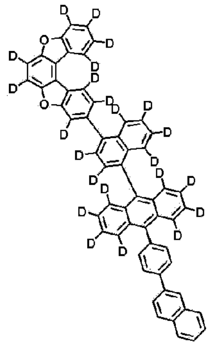
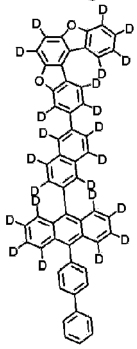
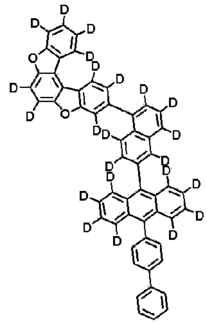
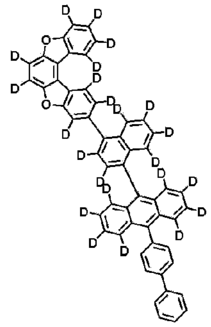
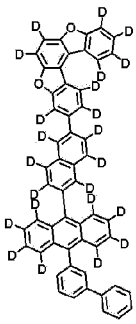
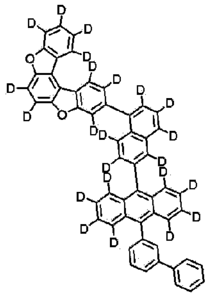
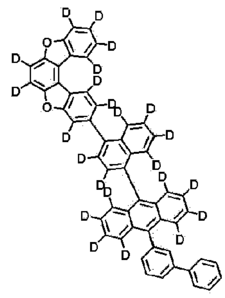
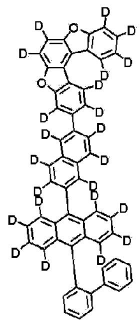
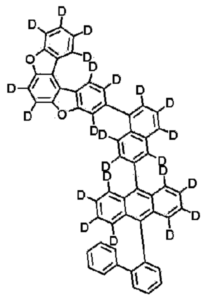
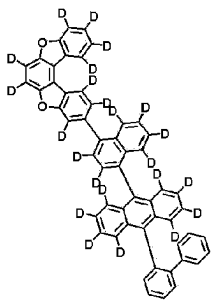
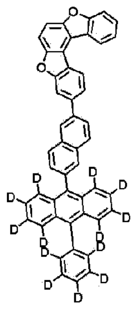
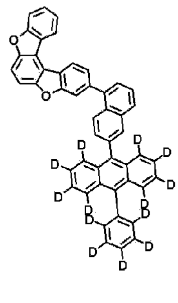
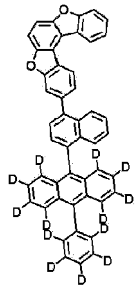

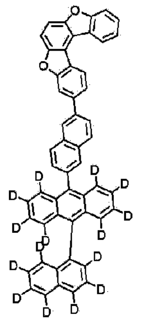
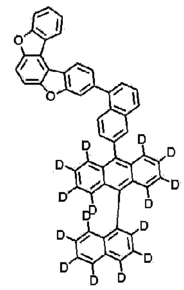
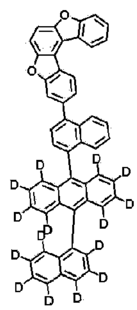

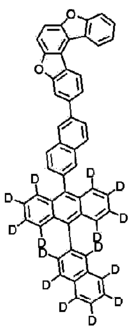
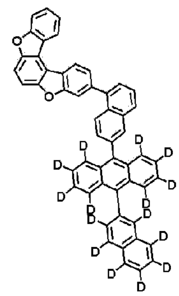
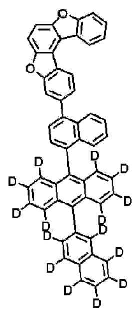

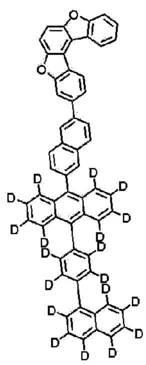
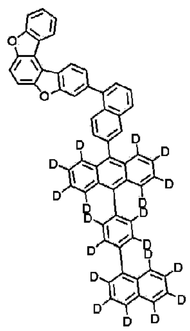
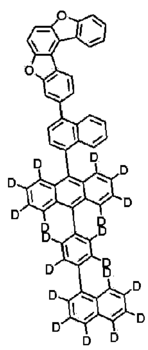

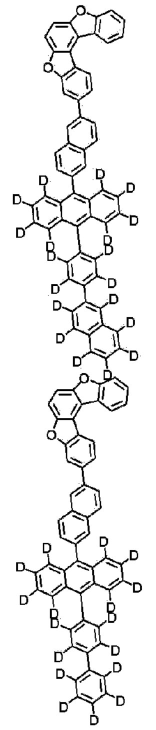
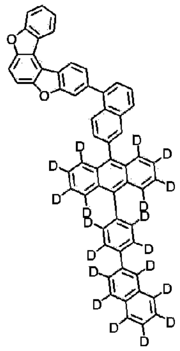
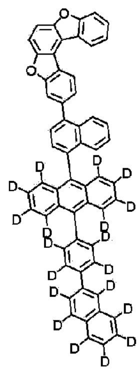
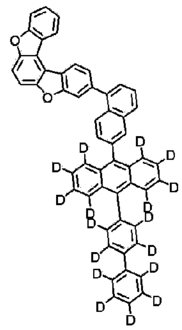
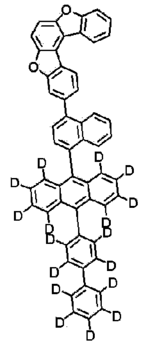
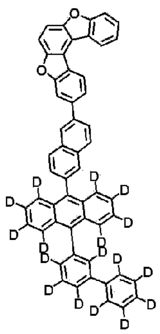
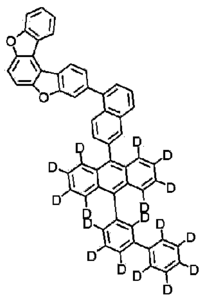
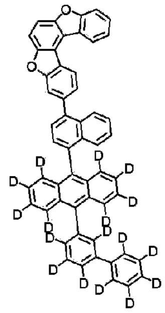
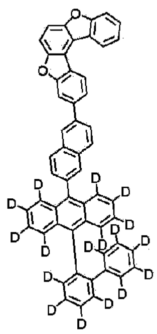
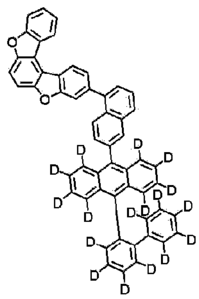
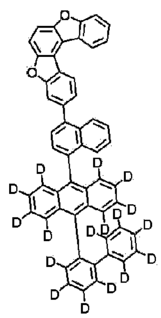

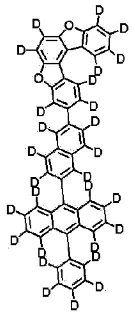
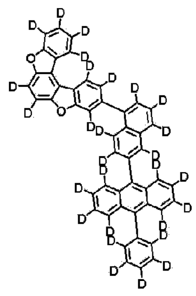
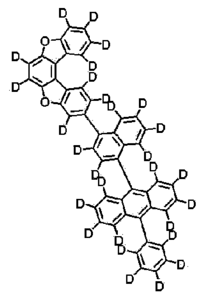

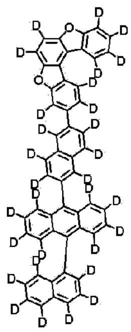
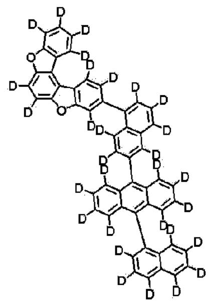
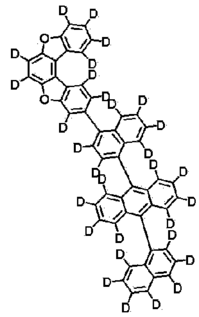

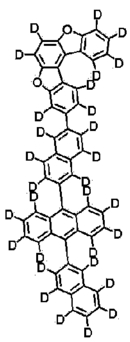
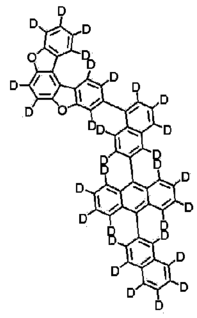
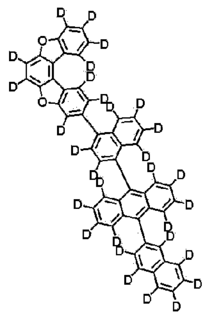

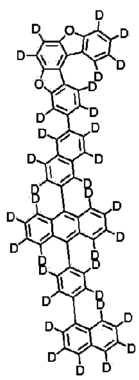
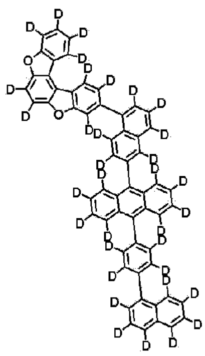
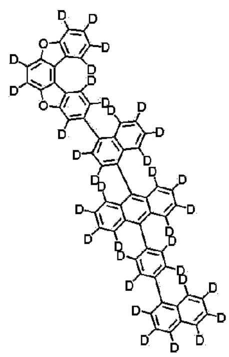

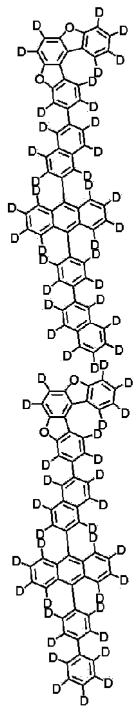
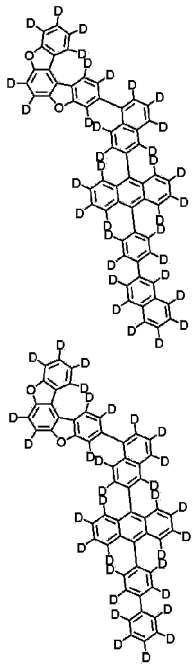
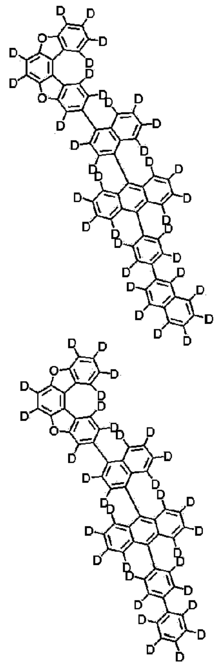

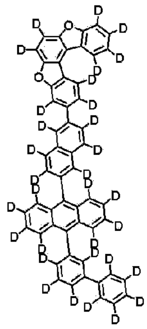
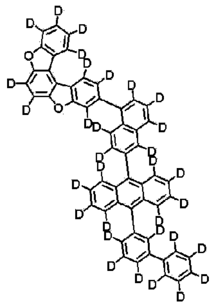
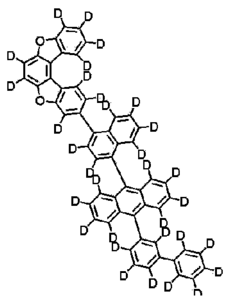

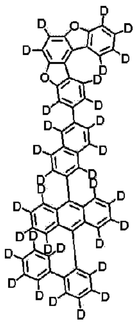
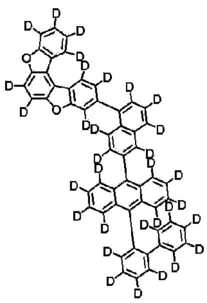
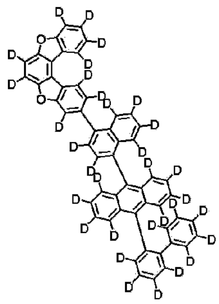

**6.** An organic light emitting device comprising:

a first electrode;
a second electrode; and
an organic material layer having one or more layers provided between the first electrode and the second electrode,
wherein the one or more layers of the organic material layer comprise the compound of any one of claims 1 to 5.

**7.** The organic light emitting device of claim 6, wherein the organic material layer comprises a hole injection layer, a hole transport layer, or an electron blocking layer, and the hole injection layer, the hole transport layer, or the electron

blocking layer comprises the compound.

8. The organic light emitting device of claim 6, wherein the organic material layer comprises a light emitting layer, and the light emitting layer comprises the compound.

9. The organic light emitting device of claim 6, wherein the organic material layer comprises a light emitting layer, and the light emitting layer comprises the compound as a host of the light emitting layer.

10. The organic light emitting device of claim 9, wherein the light emitting layer comprises a dopant, and the dopant comprises one or more selected from the group consisting of a fluorescent dopant, a phosphorescent dopant, and a thermally delayed fluorescent dopant.

11. The organic light emitting device of claim 10, wherein the fluorescent dopant comprises one or more selected from the group consisting of an arylamine-based compound and a boron-based compound.

**Patentansprüche**

1. Verbindung mit der folgenden chemischen Formel 1:

[Chemische Formel 1]

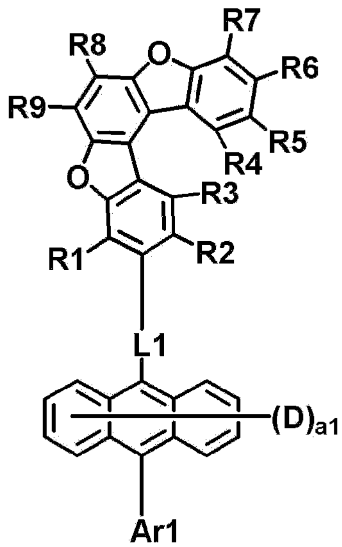

worin, in der chemischen Formel 1,
D Deuterium ist,
L1 eine Naphthylengruppe ist, die unsubstituiert oder mit Deuterium substituiert ist,
Ar1 eine substituierte oder unsubstituierte Arylgruppe ist,
R1 bis R9 gleich oder verschieden sind und jeweils unabhängig Wasserstoff oder Deuterium sind, und
a1 eine ganze Zahl von 1 bis 8 ist.

2. Verbindung nach Anspruch 1, wobei die chemische Formel 1 durch die folgende chemische Formel 1-2 dargestellt ist:

[Chemische Formel 1-2]

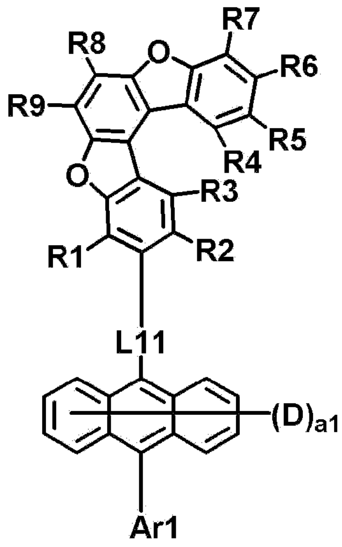

in der chemischen Formel 1-2,
sind die Definitionen von D, Ar1, a1 und R1 bis R9 die gleichen wie in der chemischen Formel 1 definiert, und L11 ist eine Naphthylengruppe, die unsubstituiert oder mit Deuterium substituiert ist.

**3.** Verbindung nach Anspruch 1, wobei die chemische Formel 1 durch die folgende chemische Formel 1-4 dargestellt ist:

[Chemische Formel 1-4]

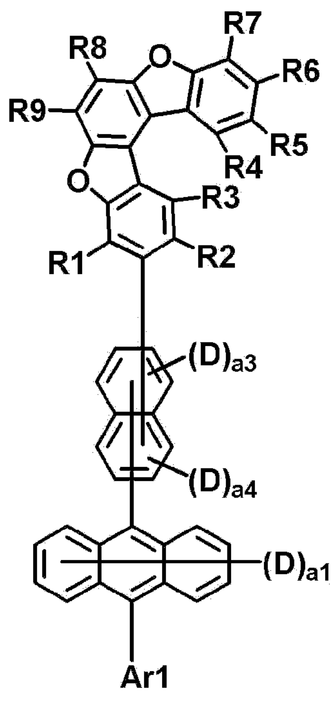

in der chemischen Formel 1-4,
sind die Definitionen von D, Ar1, a1 und R1 bis R9 die gleichen wie in der chemischen Formel 1,
a3 ist eine ganze Zahl von 0 bis 4,
a4 ist eine ganze Zahl von 0 bis 4, und

$$0 \leq a3 + a4 \leq 6.$$

4. Verbindung nach Anspruch 1, wobei Ar1 eine monocyclische oder polycyclische Arylgruppe mit 6 bis 30 Kohlenstoffatomen ist, die unsubstituiert oder mit einem oder mehreren Substituenten aus Deuterium, einer monocyclischen oder polycyclischen Arylgruppe mit 6 bis 30 Kohlenstoffatomen oder einer Kombination davon substituiert ist.

5. Verbindung nach Anspruch 1, wobei die chemische Formel 1 eine beliebige der folgenden Verbindungen ist:

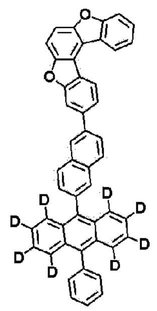
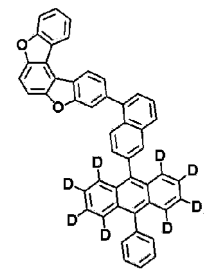
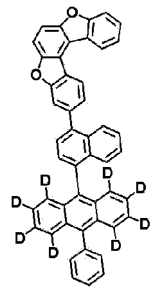
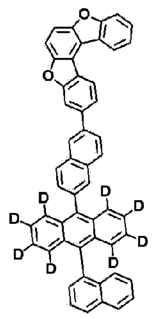
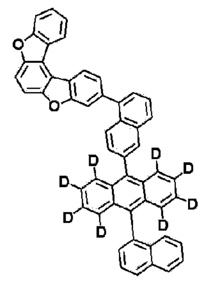
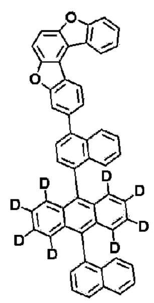
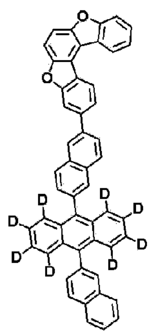
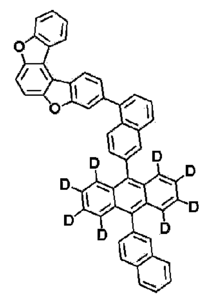
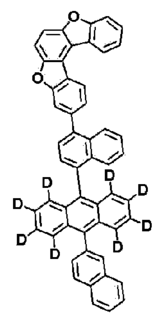
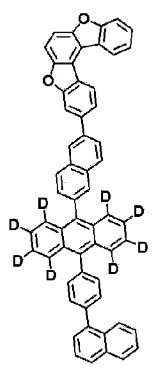
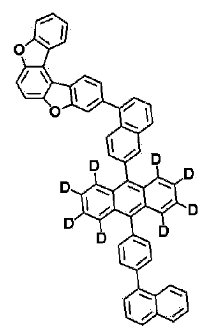
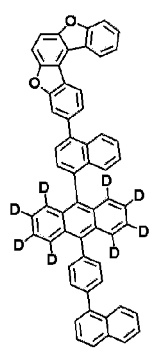

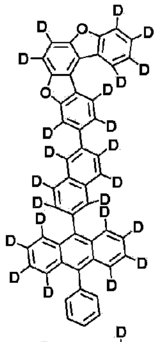
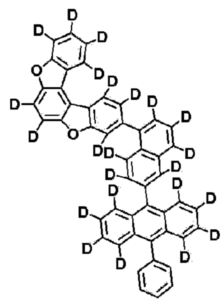
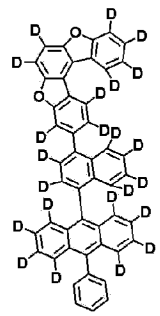
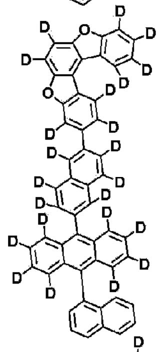
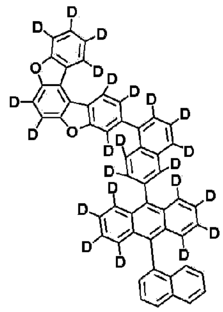
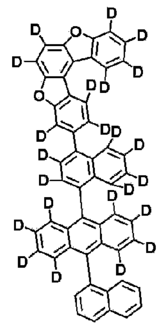
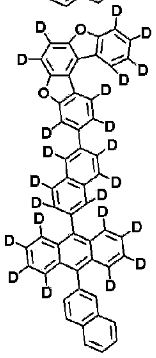
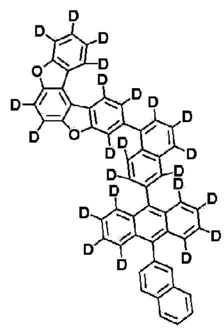
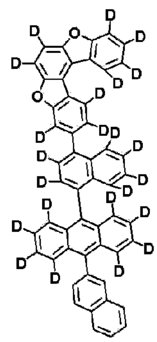
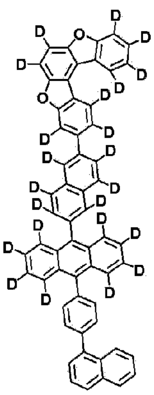
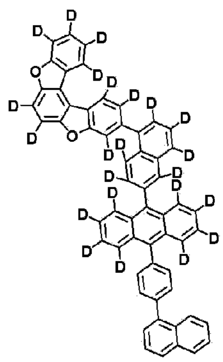
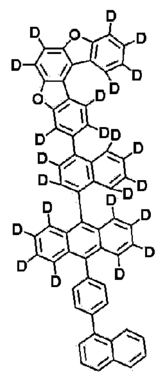

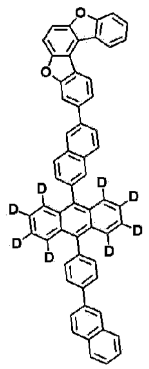
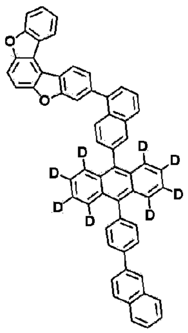
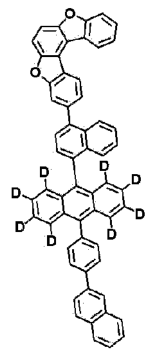
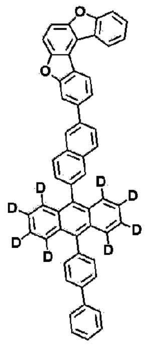
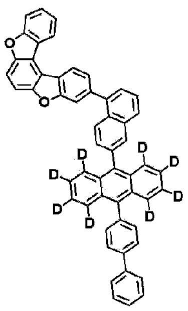
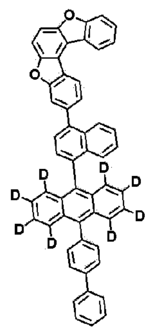
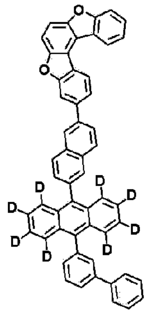
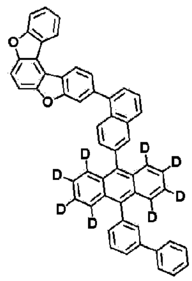
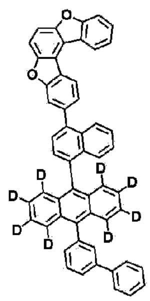
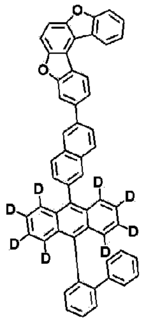
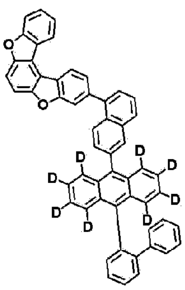
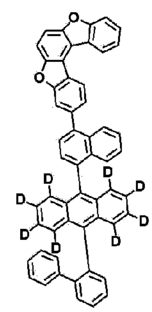

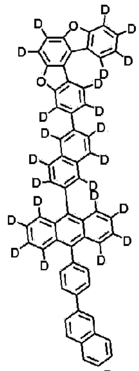
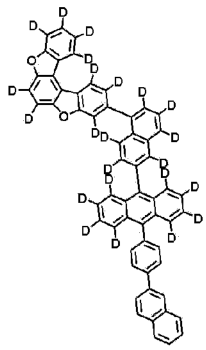
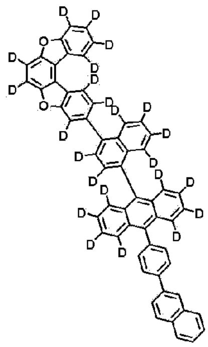

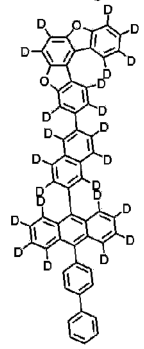
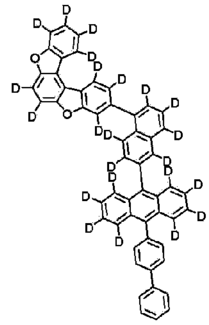
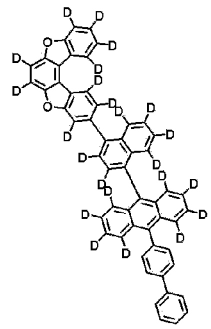

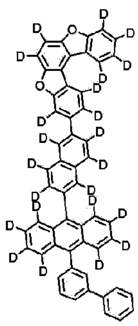
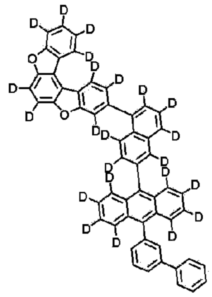
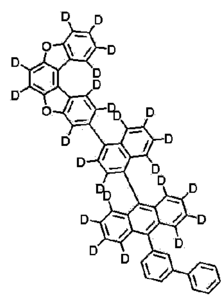

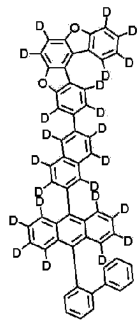
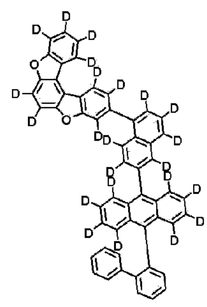
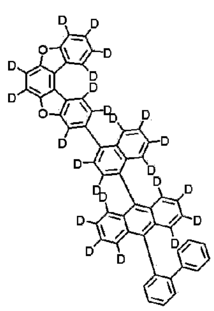

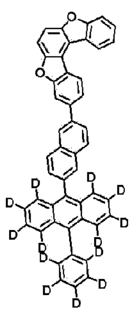
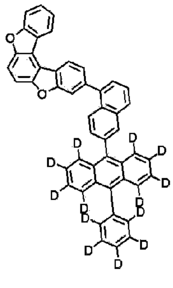
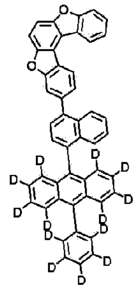

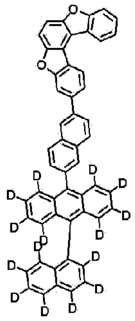

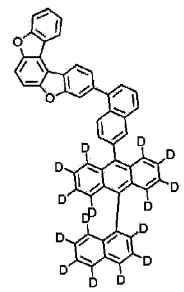

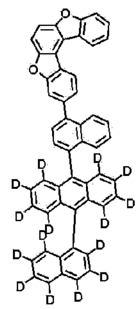

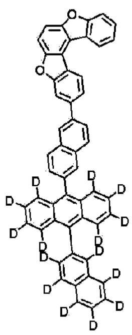

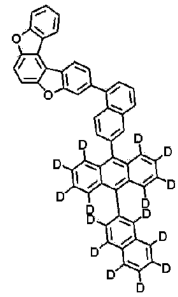

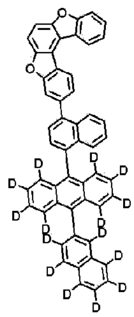

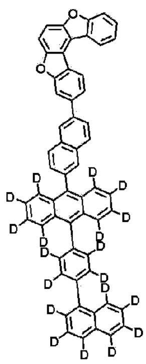

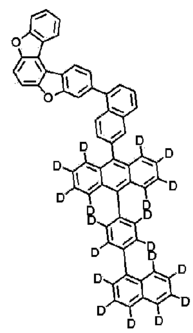

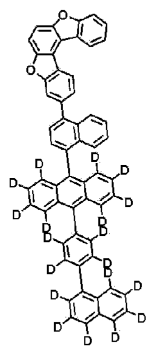

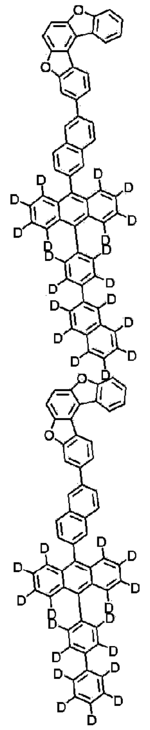

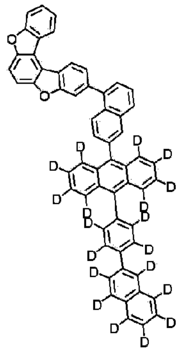

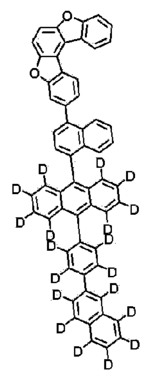

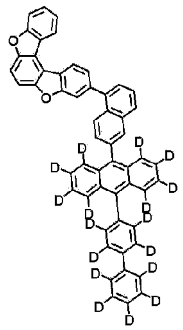

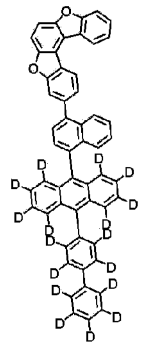

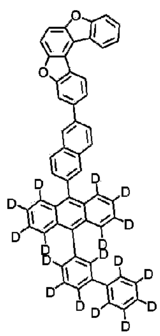

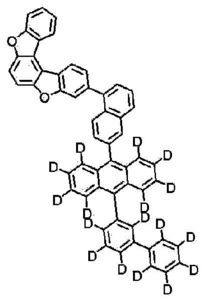

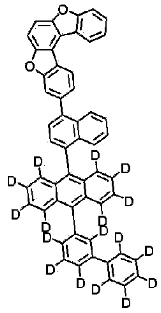

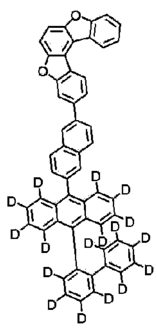

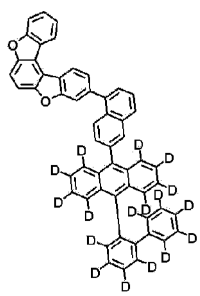

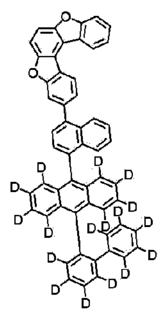

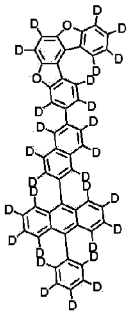

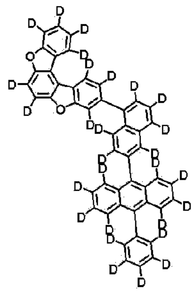

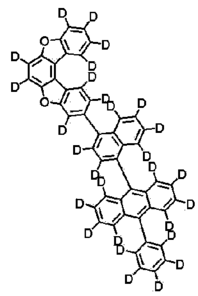

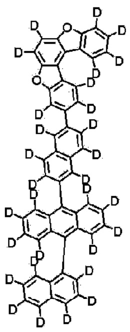

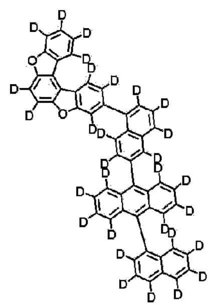

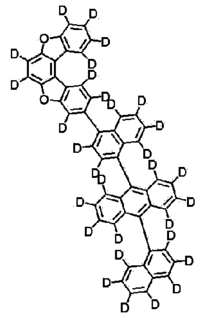

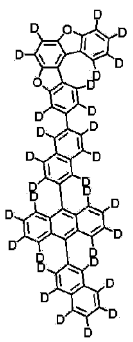

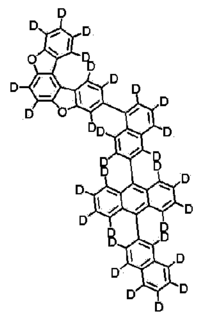

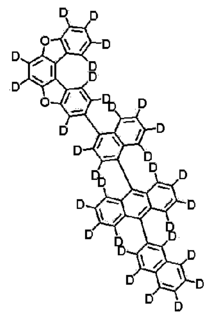

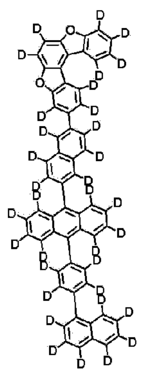

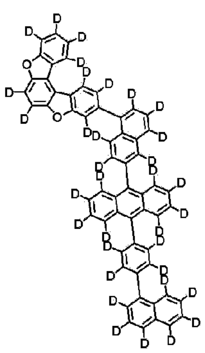

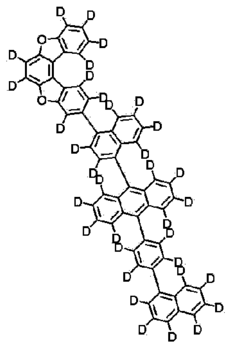

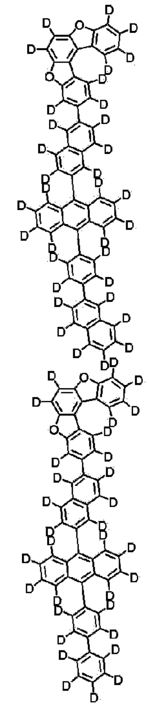
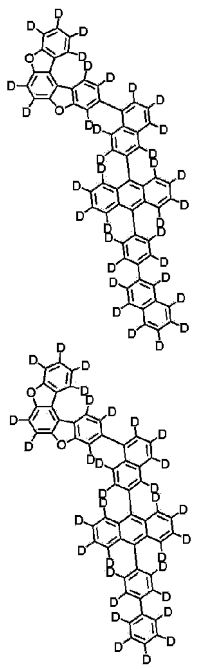
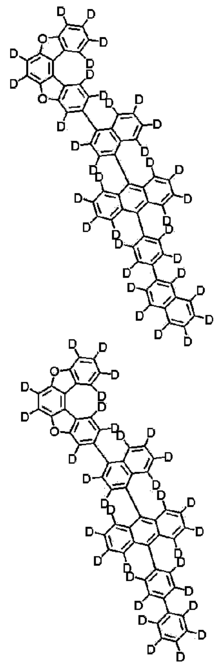

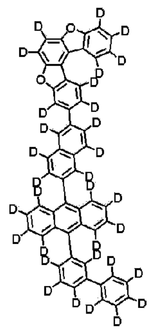
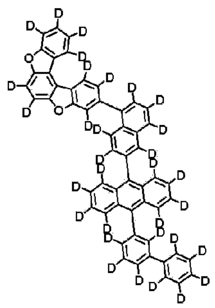
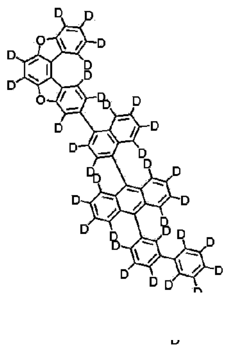

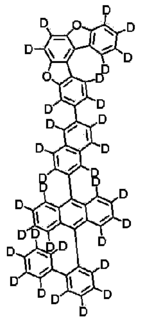
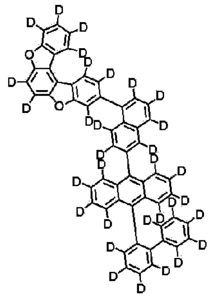
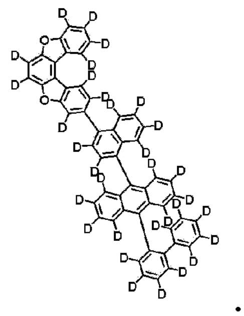

**6.** Organische Licht-emittierende Vorrichtung, umfassend:

eine erste Elektrode;
eine zweite Elektrode; und
eine organische Materialschicht mit einer oder mehreren Schichten, die zwischen der ersten Elektrode und der zweiten Elektrode angeordnet sind,
wobei die eine oder mehreren Schichten der organischen Materialschicht die Verbindung nach mindestens einem der Ansprüche 1 bis 5 umfassen.

**7.** Organische Licht-emittierende Vorrichtung nach Anspruch 6, wobei die organische Materialschicht eine Lochinjektionsschicht, eine Lochtransportschicht oder eine Elektronenblockierschicht umfasst, und die Lochinjektionsschicht, die Lochtransportschicht oder die Elektronenblockierschicht die Verbindung umfasst.

**8.** Organische Licht-emittierende Vorrichtung nach Anspruch 6, wobei die organische Materialschicht eine Licht-emittierende Schicht umfasst, und die Licht-emittierende Schicht die Verbindung umfasst.

**9.** Organische Licht-emittierende Vorrichtung nach Anspruch 6, wobei die organische Materialschicht eine Licht-emittierende Schicht umfasst, und die Licht-emittierende Schicht die Verbindung als Wirtsmaterial der Lichtemittierenden Schicht umfasst.

**10.** Organische Licht-emittierende Vorrichtung nach Anspruch 9, wobei die Licht-emittierende Schicht ein Dotierungsmittel umfasst, und das Dotierungsmittel eines oder mehrere, ausgewählt aus der Gruppe bestehend aus einem fluoreszierenden Dotierungsmittel, einem phosphoreszierenden Dotierungsmittel und einem thermisch verzögertfluoreszierenden Dotierungsmittel, umfasst.

**11.** Organische Licht-emittierende Vorrichtung nach Anspruch 10, wobei das fluoreszierende Dotierungsmittel eines oder mehrere, ausgewählt aus der Gruppe bestehend aus einer Verbindung auf Arylaminbasis und einer Verbindung auf Borbasis, umfasst.

## Revendications

**1.** Composé représenté par la formule chimique 1 suivante :

[Formule chimique 1]

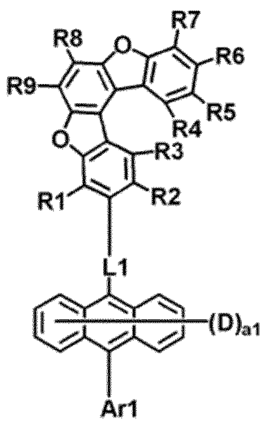

dans lequel, dans la formule chimique 1,
D est du deutérium,
L1 est un groupe naphthylène non substitué ou substitué par du deutérium,
Ar1 est un groupe aryle substitué ou non substitué,
R1 à R9 sont identiques les uns aux autres ou différents les uns des autres, et sont chacun indépendamment un hydrogène ; ou du deutérium, et
a1 est un nombre entier de 1 à 8.

**2.** Composé selon la revendication 1, dans lequel la formule chimique 1 est représentée par la formule chimique 1-2 suivante :

[Formule chimique 1-2]

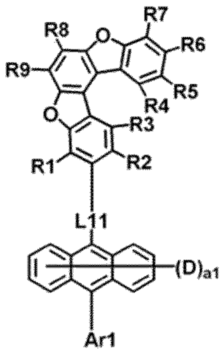

dans la formule chimique 1-2,
les définitions de D, Ar1, a1 et R1 à R9 sont les mêmes que celles définies dans la formule chimique 1, et
L11 est un groupe naphthylène non substitué ou substitué par du deutérium.

**3.** Composé selon la revendication 1, dans lequel la formule chimique 1 est représentée par la formule chimique 1-4 suivante :

[Formule chimique 1-4]

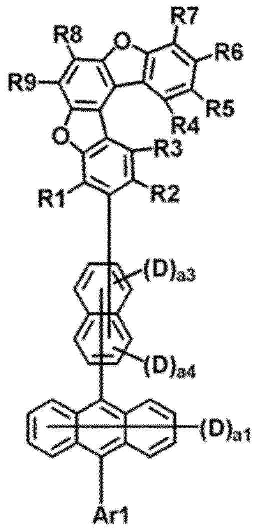

dans la formule chimique 1-4,
les définitions de D, Ar1, a1 et R1 à R9 sont les mêmes que celles définies dans la formule chimique 1,
a3 est un nombre entier de 0 à 4,
a4 est un nombre entier de 0 à 4, et

$$0 \leq a3 + a4 \leq 6.$$

**4.** Composé selon la revendication 1, dans lequel Ar1 est un groupe aryle monocyclique ou polycyclique présentant 6 à 30 atomes de carbone, qui est non substitué ou substitué par un ou plusieurs substituants de deutérium, un groupe aryle monocyclique ou polycyclique présentant 6 à 30 atomes de carbone, ou une de leurs combinaisons.

**5.** Composé selon la revendication 1, dans lequel la formule chimique 1 est l'une quelconque sélectionnée parmi les composés suivants :

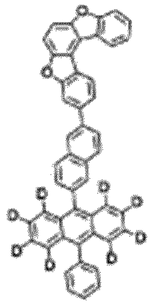
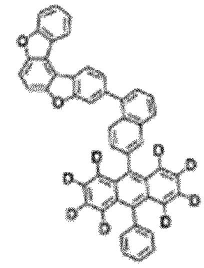
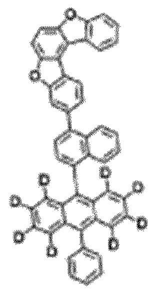
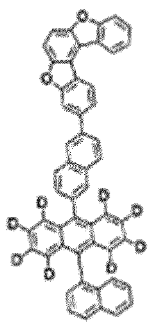
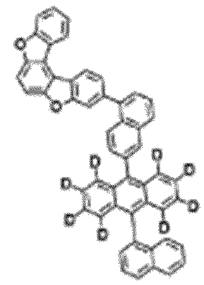
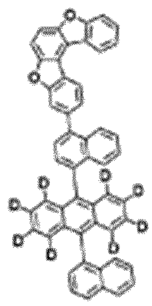
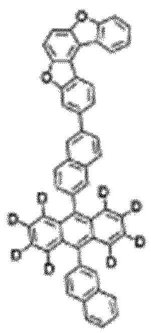
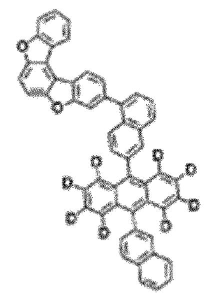
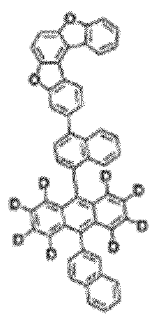
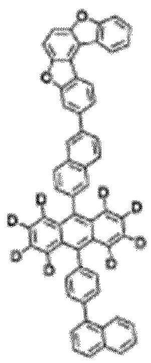
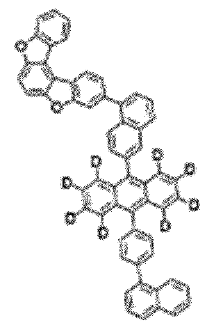
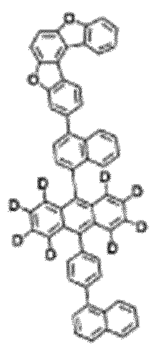

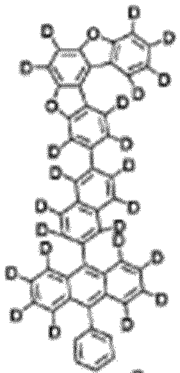

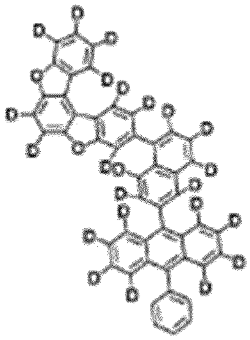

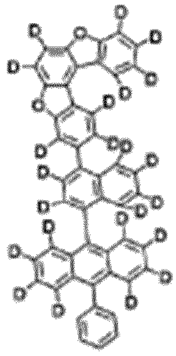

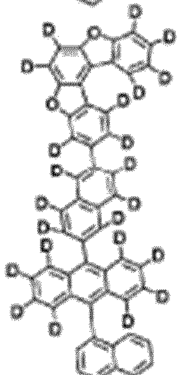

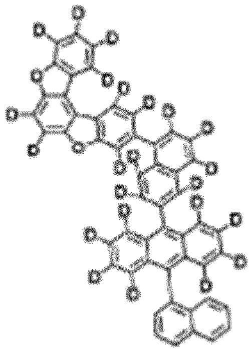

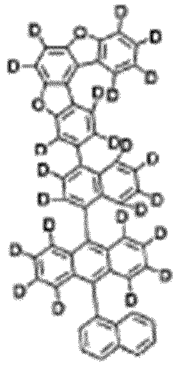

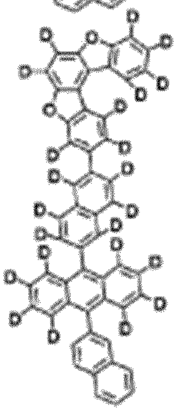

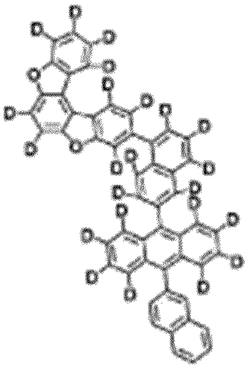

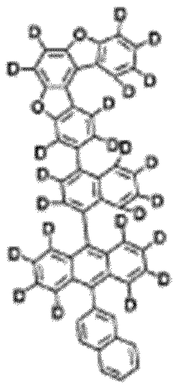

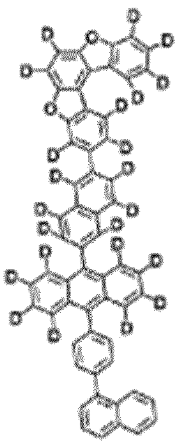

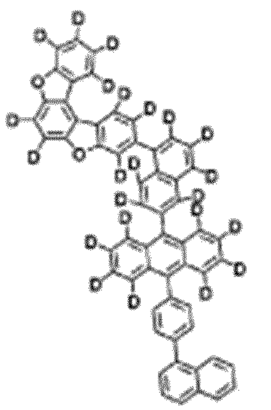

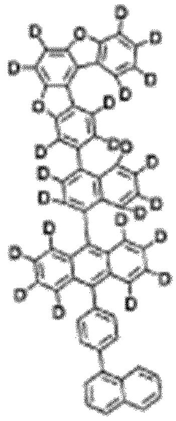

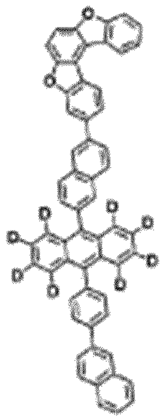
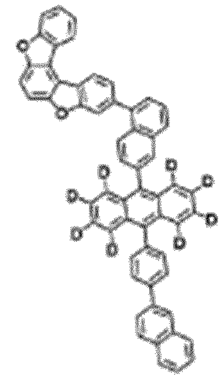
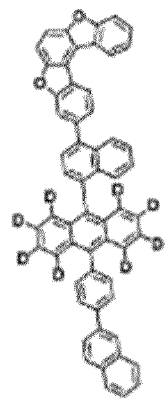

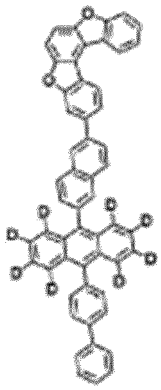
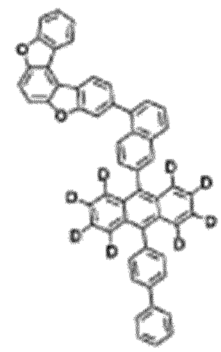
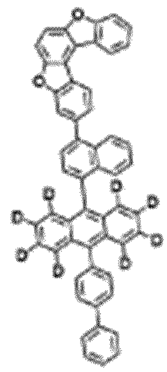

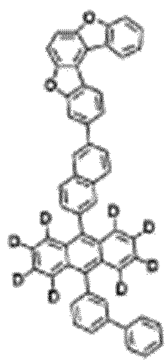
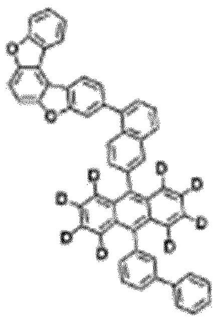
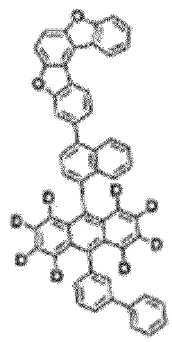

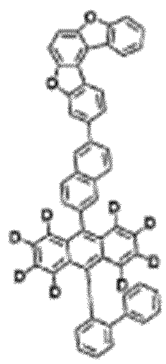
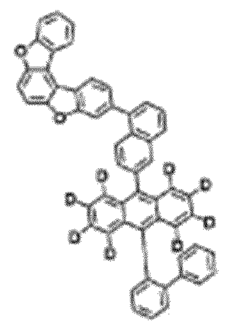
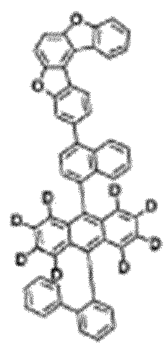

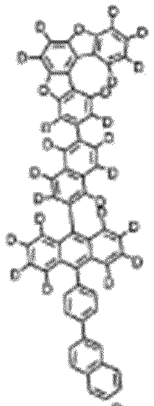
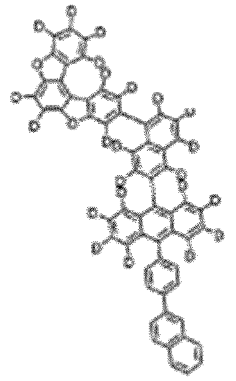
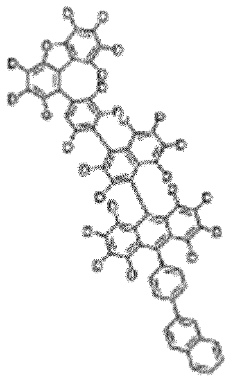
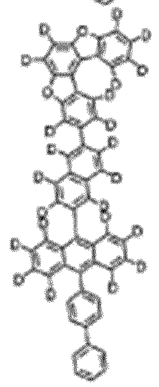
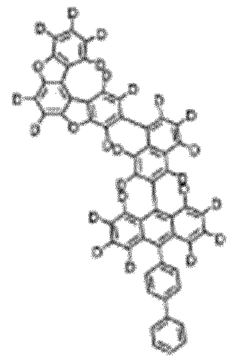
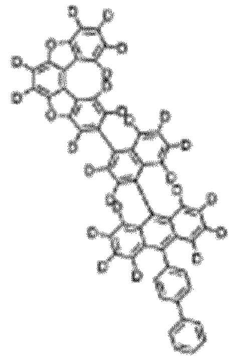
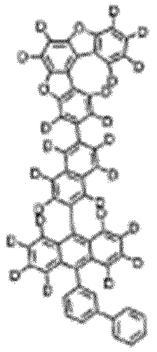
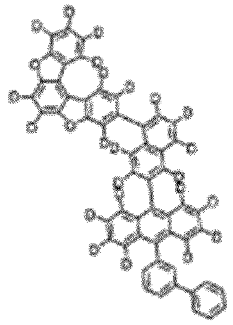
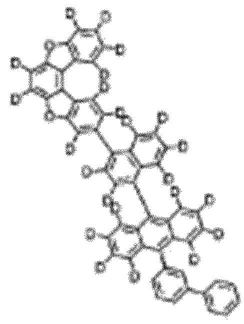
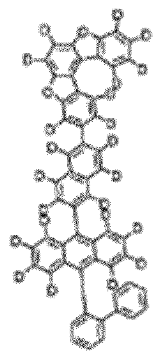
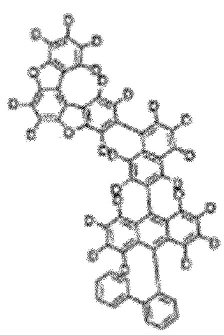
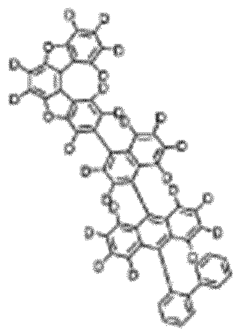

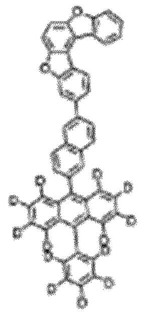
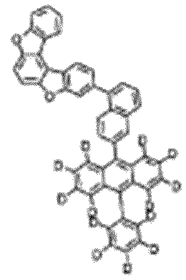
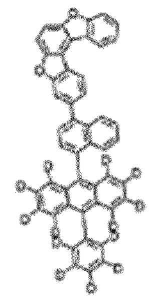

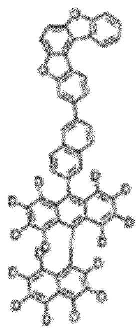
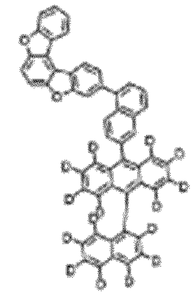
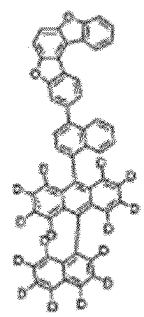

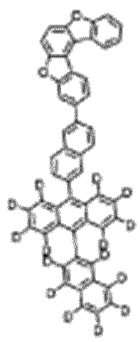
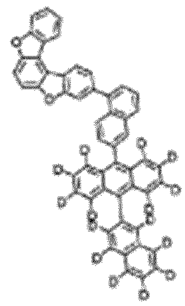
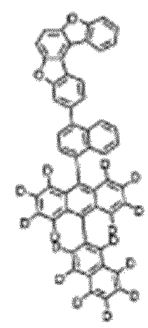

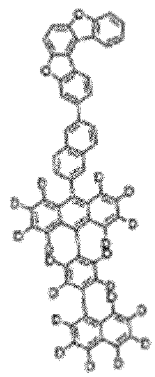
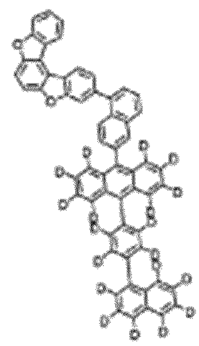
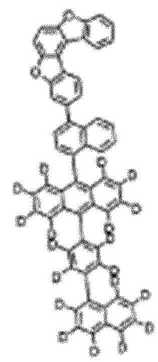

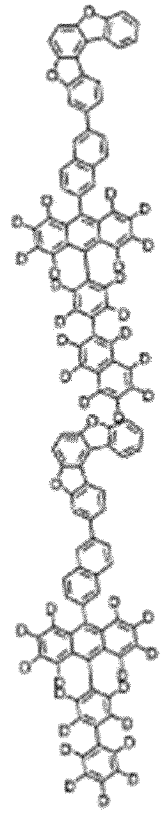

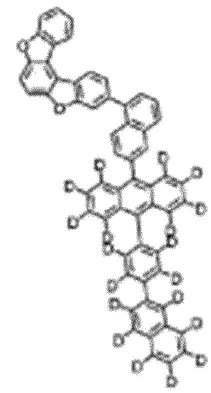

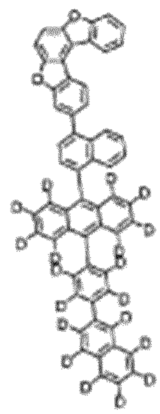

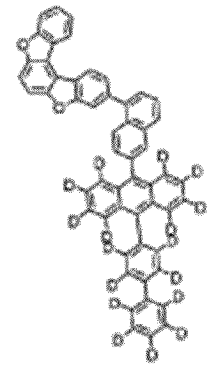

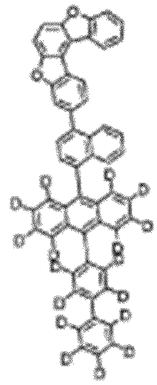

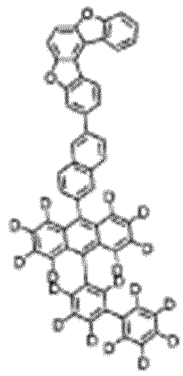

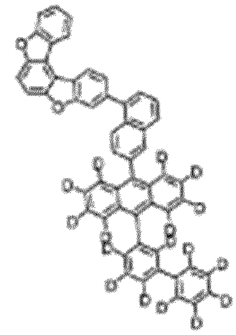

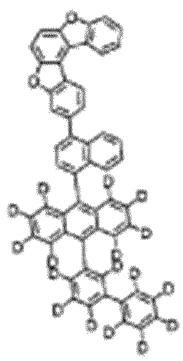

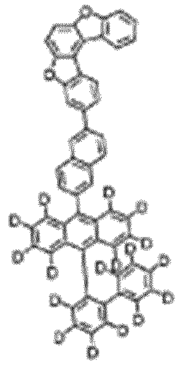

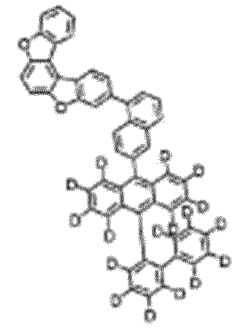

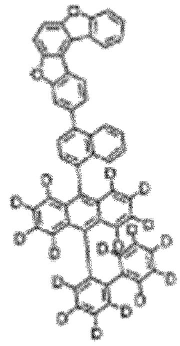

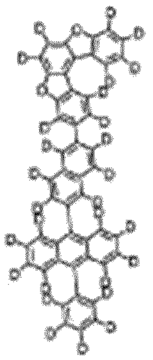

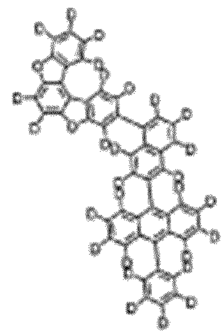

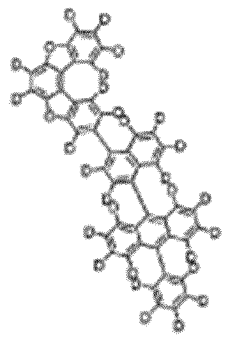

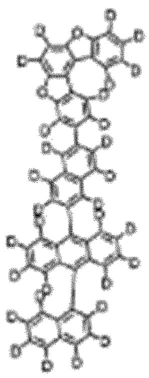

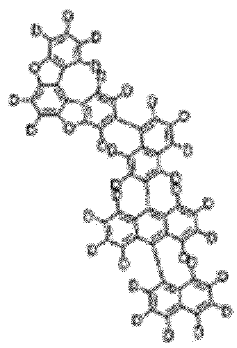

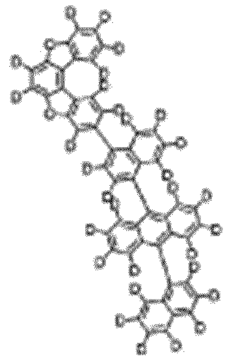

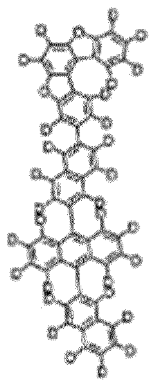

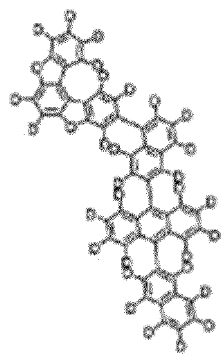

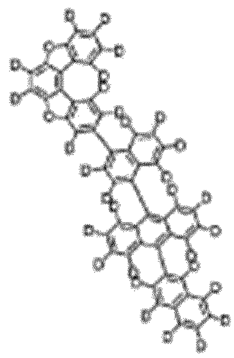

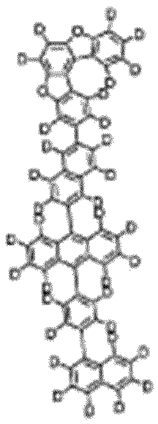

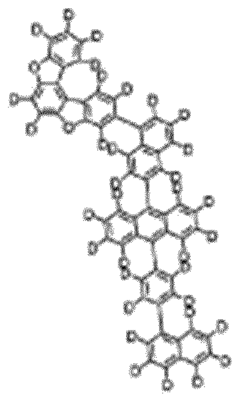

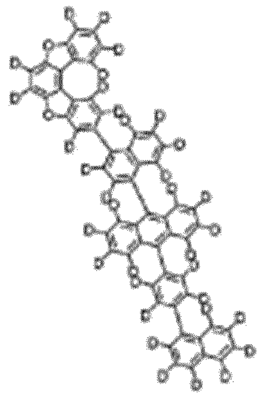

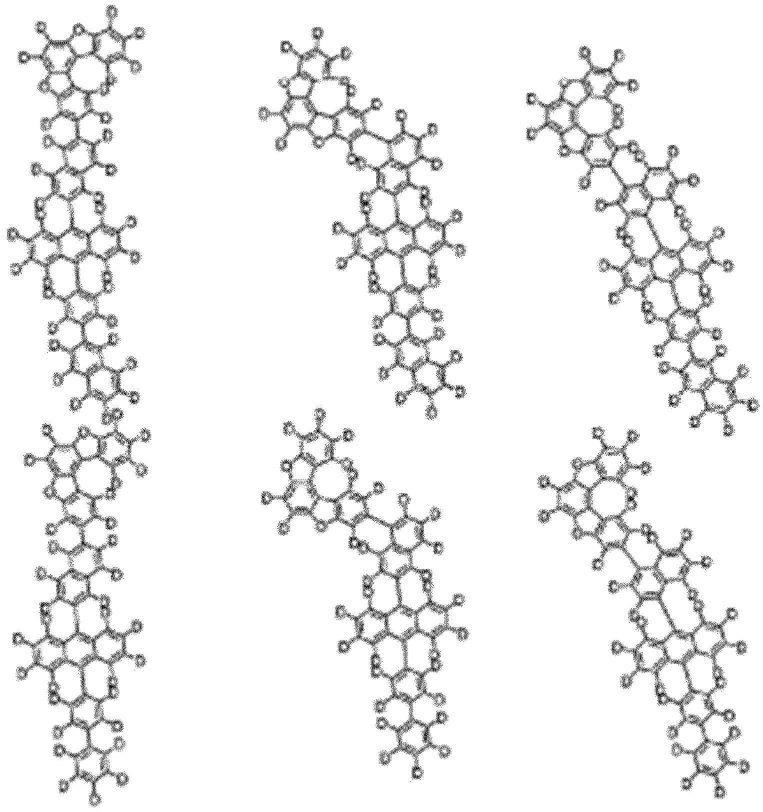

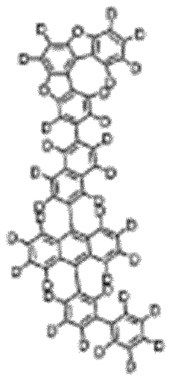

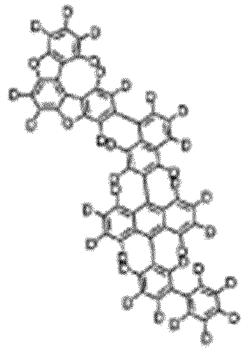

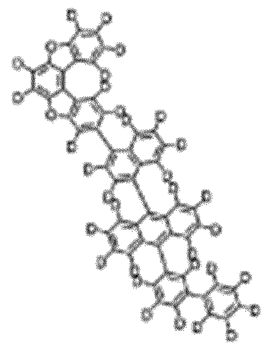

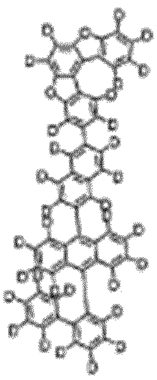

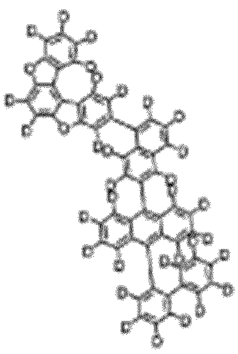

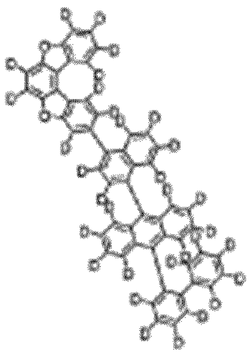

**6.** Dispositif électroluminescent organique comprenant :

une première électrode ;

une seconde électrode ; et
une couche de matériau organique présentant une ou plusieurs couches prévues entre la première électrode et la seconde électrode,
dans lequel une ou plusieurs couches de la couche de matériau organique comprennent le composé selon l'une quelconque des revendications 1 à 5.

**7.** Dispositif électroluminescent organique selon la revendication 6, dans lequel la couche de matériau organique comprend une couche d'injection à trous, une couche de transport à trous, ou une couche de blocage d'électrons, et la couche d'injection à trous, la couche de transport à trous, ou la couche de blocage d'électrons comprend le composé.

**8.** Dispositif électroluminescent organique selon la revendication 6, dans lequel la couche de matériau organique comprend une couche électroluminescente et la couche électroluminescente comprend le composé.

**9.** Dispositif électroluminescent organique selon la revendication 6, dans lequel la couche de matériau organique comprend une couche électroluminescente et la couche électroluminescente comprend le composé en tant qu'hôte de la couche électroluminescente.

**10.** Dispositif électroluminescent organique selon la revendication 9, dans lequel la couche électroluminescente comprend un dopant, et le dopant comprend un ou plusieurs éléments sélectionnés parmi le groupe consistant en un dopant fluorescent, un dopant phosphorescent et un dopant fluorescent à retardement thermique.

**11.** Dispositif électroluminescent organique selon la revendication 10, dans lequel le dopant fluorescent comprend un ou plusieurs éléments sélectionnés parmi le groupe consistant en un composé à base d'arylamine et un composé à base de bore.

[Figure 1]

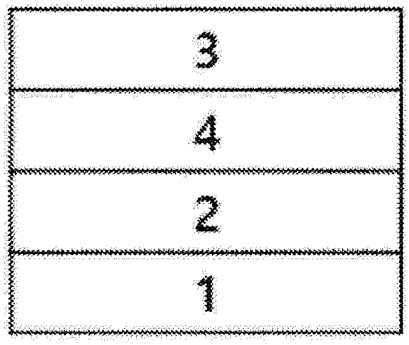


[Figure 2]

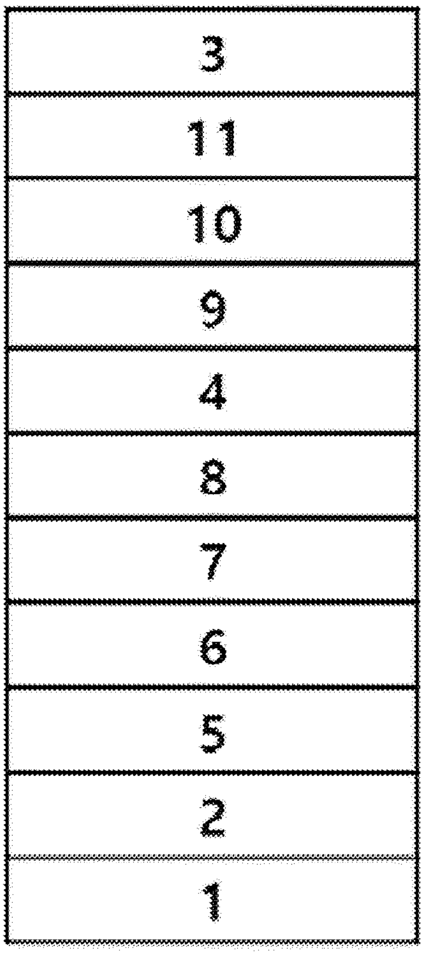

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020210116104
- KR 1020210093625
- WO 2021150090 A1